# EUROPEAN PATENT SPECIFICATION

(11) **EP 4 574 192 B1**
(45) Date of publication and mention of the grant of the patent: **04.11.2026**
(21) Application number: 25162160.3
(22) Date of filing: 06.07.2017
(51) Int. Cl.: A61M 16/06

(54) **RESPIRATORY INTERFACE**
RESPIRATORISCHE SCHNITTSTELLE
INTERFACE RESPIRATOIRE

(30) Priority: 06.07.2016 US 201662358731 P; 25.10.2016 US 201662412399 P; 22.12.2016 US 201662438207 P
(43) Date of publication of application: 25.06.2025
(62) Document number(s) of the earlier application(s) in accordance with Art. 76 EPC: 17823742.6 / 3 481 478
(73) Proprietor: Fisher & Paykel Healthcare Limited, East Tamaki, Auckland 2013 (NZ)
(72) Inventor: HOCKING, Jake Baker, 2013 Auckland (NZ); MCLAREN, Mark Arvind, 2013 Auckland (NZ); SPEAR, Tony William, 2013 Auckland (NZ); BORNHOLDT, Melissa Catherine, 2013 Auckland (NZ)
(74) Representative: Forresters IP LLP

(56) References cited:
- WO-A1-2014/045245
- AU-A1- 2014 305 231
- US-A1- 2015 335 846
- US-B1- 7 793 987

## Description

### BACKGROUND OF THE INVENTION

### Field of the Invention

The invention generally relates to respiratory interfaces and in particular but not exclusively to respiratory interfaces comprising magnetic elements for coupling and/or adjusting parts of the interface.

### Description of the Related Art

Respiratory interfaces or masks are used to provide respiratory gas or gases, such as air in CPAP therapy, including in for example VPAP and BiPAP systems, or NIV, or high flow rate therapy, for example.

Commonly a respiratory interface comprises a mask frame to which for example headgear attaches which holds the interface in position on the user's head when worn, and a seal module (sometimes also referred to as a cushion or cushion module) configured to couple to the mask frame and interface to a user's mouth and/or nose to deliver respiratory gases to the user. A respiratory interface may comprise a nasal, oral, or oro-nasal (also referred to as full face) seal module. In turn an interface may be an indirect interface which covers the nose, mouth, or both, or a direct interface such as an interface comprising nasal nozzles or pillows or similar which enter into and seal against or within the nares of the wearer or cannula which non-sealingly enter the nares. The seal module can be formed entirely or almost entirely of a soft material which is comfortable against the wearer's face, such as commonly a silicone material, or the seal module may comprise a rigid or semi-rigid frame interfacing part formed of a rigid or semi-rigid material and which couples to the mask frame and a seal part formed of a relatively soft material.

Typically the seal module couples mechanically to the mask frame via a friction fit, snap fit, or other interference fit, of formations on the seal module and mask frame to one another. The seal module couples detachably to the mask frame so that it can be removed for cleaning or so that different sizes of seal module may interface with the mask frame. Prior art arrangements are known from WO2014/045245, which discloses a respiratory mask having a magnetically supported cushion, US 2015/0335846, which discloses a patient interface, and US 7793987, which discloses a magnetic coupling assembly.

### SUMMARY OF THE INVENTION

The invention is defined by the appended claims. Subject-matter referred to as embodiments, arrangements, aspects and/or disclosures which do not fall under the scope of the claims are not part of the invention.

### Seal module to frame magnetic connection

In broad terms in one aspect the disclosure comprises a respiratory interface comprising:
a mask frame,
a seal module configured to couple to the mask frame and interface to a user's mouth and/or nose to deliver respiratory gases to the user, and
a magnetic coupling system arranged to magnetically couple the seal module to the mask frame. Magnetic coupling between a rear, user facing, side of the mask frame and a forward side of the seal module potentially enables a method of use where the seal module can be removed while headgear remains in place on the user. Preferably the magnetic coupling system comprises multiple magnets associated with each of the seal module or mask frame. Preferably, magnets are provided to the seal module and mask frame such that they attract only when the seal module is coupled to the mask frame in a predetermined orientation of the seal module relative to the mask frame and repel when the seal module is offered to the mask frame in any orientation other than said predetermined orientation. Multiple magnets may each comprise a segment of solid (non-particulate) magnetic material or the magnet or magnets comprise particulate magnetic material. In one form the particulate magnetic is material embedded in soft material of the seal module.

In broad terms in another aspect the disclosure comprises a seal module for a respiratory interface, the seal module configured to couple to a mask frame and interface to a user's mouth and/or nose to deliver respiratory gases to the user, and comprising at least one magnet or ferrous part associated with the seal module to magnetically couple the seal module to the mask frame.

In one form the respiratory interface may include magnets associated with the seal module provided at left and right sides of the seal module and magnets associated with the mask frame are provided at left and right sides of the mask frame. Preferably, magnets associated with the seal module or mask frame are provided to the seal module or mask frame with movement in a direction in which the seal module and mask frame interface so that on offering together the seal module and mask frame magnets associated with the seal module or mask frame move toward the magnets associated with the mask frame or seal module. Preferably the seal module is formed of a soft material. The seal module may comprise a frame interfacing part formed of a rigid or semi-rigid material and which couples to the mask frame and a seal part formed of a relatively soft material. The seal module may be an indirect or direct nasal seal module or an oro-nasal seal module.

### Magnetic hold of nasal bridge compliance adjustment

In broad terms in another aspect the disclosure comprises a seal for a respiratory interface, the seal module configured to interface to a user's mouth and/or nose to deliver respiratory gases to the user, and comprising at least one magnet or ferrous part associated with the seal to magnetically hold a first part of the seal in position relative to another part of the seal or a part of a frame of the respiratory interface. Preferably multiple magnets or ferrous parts associated with the seal or a frame to magnetically hold a first part of the seal in multiple selectable adjustment positions of the first part of the seal relative to another part of the seal or a part of a frame of the respiratory interface. Preferably, the seal comprises a frame interfacing part formed of a rigid or semi-rigid material and which couples to a mask frame and a seal part formed of a relatively soft material. In one form the magnet or magnets comprise particulate magnetic material, that is preferably embedded in soft material of the seal module.

In broad terms in another aspect the disclosure comprises a respiratory interface comprising a mask frame and a seal as above.

### Embedded particulate magnetic material

In broad terms in another aspect the disclosure comprises a respiratory interface and/or headgear, comprising a first part and a second part which in use are coupled together, wherein at least one of the first part and second part comprise a magnet or magnets for magnetic coupling of the first part and second part together which comprise a particulate magnetic material embedded in the first part and/or second part. In one form the particulate magnetic material is embedded in the first part and/or second part by moulding. Preferably, one of the first part and second part is a seal module and the particulate magnetic material is embedded in soft material of the seal module.

### Nasal to full face magnetically connected seal

In broad terms the invention comprises a respiratory interface comprising: a first seal part configured to interface to one of a user's mouth and nose to deliver respiratory gases to the user, and a second seal part configured to interface to the other of a user's mouth and nose to also deliver respiratory gases to the user, and a magnetic coupling system arranged to magnetically couple the first and second seal parts to one another, optionally to a common mask frame and so that at least one of the first and second seal parts is detachable from the other or from common mask frame.

Preferably, at least one magnet associated with one of the first or second seal parts or the mask frame and at least one magnet associated with another of the first or second seal parts or the mask frame are provided to the seal parts or mask frame such that they attract when the seal parts are coupled to each other or to the mask frame in a predetermined correct orientation and repel in any orientation other than said predetermined correct orientation.

### Magnet mouth connection

In broad terms in another aspect the disclosure comprises a respiratory interface comprising:
a mask configured to interface to a user's mouth and/or nose to deliver respiratory gases to the user,
an oral insert configured to be retained in the user's mouth when the mask is worn, and
a magnetic coupling system arranged to magnetically couple the mask frame and oral insert to at least assist in retaining the mask in position on the face of the user. In one form the oral insert is configured to be retained at least in part on upper teeth of the user when worn in the user's mouth. Preferably, the respiratory interface does not also comprise headgear to assist in retaining the mask in position when worn by the user.

### Magnetic conduit connection

In broad terms in another aspect the disclosure comprises a respiratory interface comprising:
a mask,
a conduit connectable to the mask or to a lead in tube to the mask to deliver respiratory gases to the user, and
a magnetic coupling system comprised of magnets associated with both of the conduit to the mask and the mask or a lead in tube to the mask, to magnetically couple the conduit to the mask or a lead in tube to the mask, wherein at least one magnet associated with one of the conduit to the mask and the mask or a lead in tube to the mask and at least one magnet associated with another of the conduit to the mask and the mask or a lead in tube to the mask are provided to the conduit and the mask or a lead in tube to the mask such that they attract when the conduit and the mask or a lead in tube to the mask are coupled to each other or to the mask frame in a predetermined correct orientation and repel in an orientation other than said predetermined correct orientation.

In broad terms in another aspect the disclosure comprises a magnetic coupling system arranged to magnetically couple a conduit to a fitting or another conduit, comprising a first annular part configured to receive a second annular part over the first annular part, comprising:
at least one magnet associated with one of the first annular part and second annular part and at least one ferrous part associated with another of the first annular part and second annular part, or
magnets associated with both of the first annular part and second annular part.

### Magnetic modular headgear

In broad terms in another aspect the disclosure comprises headgear for a respiratory interface, comprising:
a first headgear part
at least a second headgear part, and
a magnetic coupling system arranged to magnetically couple the first and second headgear parts to one another. In one form there may also be a third headgear part and wherein the magnetic coupling system is arranged to magnetically couple the first, second, and third headgear parts to one another. In a further form there may be a fourth headgear part and wherein the magnetic coupling system is arranged to magnetically couple the first, second, third, and fourth headgear parts to one another. Preferably, the first and second, and optionally third or third and fourth headgear parts, comprise at least one magnet at or towards two ends thereof. Alternatively, the first and second, and optionally third or third and fourth headgear parts, comprise at least one magnet at or towards one end thereof and at least one ferrous part at or towards another end thereof. In a further form at least two magnets associated with one with one of one of the first and second, and optionally third or third and fourth headgear parts and another with another of the first and second, and optionally third or third and fourth headgear parts, are provided to the headgear parts such that they attract when the headgear parts are coupled in a predetermined correct orientation and repel in an orientation other than said predetermined correct orientation.

### Headgear buckle (also headgear to mask buckle)

In broad terms in another aspect the disclosure comprises a headgear for a respiratory interface, or headgear and a respiratory interface together, comprising:
a first headgear part having one of a protrusion and one or more recesses at or towards an end thereof,
a second headgear part having another of a protrusion and one or more recesses at or towards an end thereof, or a mask comprising one of a protrusion and one or more recesses on a part of the mask,
a magnetic coupling system arranged to magnetically hold together when engaged to couple same to one another, the protrusion or one or more recesses of the first headgear part and the protrusion or one or more recesses of the second headgear part or mask.

### Headgear connection broken by 3rd magnet/decoupling mechanism

In broad terms in another aspect the disclosure comprises headgear for a respiratory interface, or headgear and a respiratory interface together, comprising: a first headgear part comprising a first magnet at or towards an end thereof, a second headgear part comprising a second magnet at or towards an end thereof, or a mask comprising a second magnet on a part of the mask, arranged to magnetically couple the first headgear part to the second headgear part or mask, and a decoupling tool comprising a third magnet and insertable between the first and second magnets when the first headgear part and the second headgear part or mask are coupled, to at least assist in de-coupling same.

In a further form headgear for a respiratory interface may be provided, or headgear and a respiratory interface together, comprising: a first headgear part having an inflatable portion, a second headgear part, a magnetic coupling system arranged to magnetically hold the first and second headgear parts together, a decoupling mechanism comprising a pump associated with the inflatable portion, wherein activation of the pump causes the inflatable portion to inflate, causing the first and/or second part to move away from the other part.

Preferably, the second headgear part has an inflatable portion and activation of the pump causes the inflatable portion to inflate. In one form the parts move away from each other by one or both of the parts moving from a curved configuration to a relatively straight configuration. A substantial portion of each or both of the first and second headgear parts may be inflatable. Preferably the first headgear part and the second headgear part overlap when held together by the magnetic coupling system.

In a further form the respiratory interface or headgear according to any one of the previous aspects comprises a magnet or magnets comprising a segment of solid (non-particulate) magnetic material or a magnet or magnets comprising particulate magnetic material. Preferably the particulate magnetic is material embedded in a part of the respiratory interface or headgear. This may comprise a mask frame and a separate seal module. Preferably the seal module comprises a frame interfacing part formed of a rigid or semi-rigid material and which couples to a mask frame and a seal part formed of a relatively soft material. In one form the interface may comprise an indirect nasal seal or direct nasal seal or an oro-nasal seal. A respiratory interface or headgear having features according to any aspect described herein may comprise any features/integers in combination.

### General

Interfaces of the invention may be used in continuous positive airway pressure (CPAP) systems for providing a heated and optionally also humidified air stream to a user (U) through the interface worn by the user, or alternatively in other forms of respiratory systems, such as for example VPAP (Variable Positive Airway Pressure) systems, BiPAP (Bi level Positive Airway Pressure) systems, or in non-invasive ventilation (NIV), or high flow rate (not necessarily also above ambient pressure) therapy, for example, and are described herein generally with reference to CPAP therapy by way of example only. The interfaces may be useful particularly for CPAP therapy at air pressures in the range about 0.5 to about 40 cm H₂O. However the interfaces may also be used in in systems or therapy in which the air or other gases are not heated and/or humidified.

In this specification the term "comprising" means "consisting at least in part of". When interpreting a statement in this specification and claims that includes "comprising", features other than that or those prefaced by the term may also be present. Related terms such as "comprise" and "comprises" are to be interpreted similarly.

### Brief Description of the Drawings

Embodiments are described with reference to the accompanying drawings, by way of example and without intending to be limiting, in which:
Figure 1 is a view of an embodiment of a respiratory interface comprising a mask frame and separate seal, and also showing headgear which holds the interface in position on the user's head when worn,
Figure 2 is a view of the interface of Figure 1 similar to Figure 1 (not also showing headgear), showing the mask frame and seal module separated,
Figure 3 is a front view of the seal module of the interface of Figure 1,
Figure 4 is a rear view of the mask frame of the interface of Figure 1,
Figures 5A and 5B are schematic cross-section views of interfacing parts of a seal module and mask frame of an interface of another example, separated in Figure 5A and connected in Figure 5B, and Figure 5C is a schematic exploded view of magnet parts of the seal module and mask frame and a gel pad between as will be further described,
Figure 6 is a schematic cross-section view of a seal module and the interfacing part of a mask frame of an interface of another example,
Figure 7A is a schematic cross-section view of a seal module and the interfacing part of a mask frame of an interface of another example, and Figures 7B and 7C are schematic views of magnet parts of the seal module and mask frame in correct and incorrect orientations relative to one another respectively,
Figures 8A, 8B, and 8C are schematic cross-section views of interfacing parts of a seal module and mask frame of an interface of a further example, connected in Figure 8A, near connected in Figure 8B, and apart in Figure 8C,
Figures 9A and 9B are schematic views of an embodiment of a respiratory interface in which magnets associated with the seal module and mask frame are provided at left and right sides of the seal module and mask frame,
Figure 10 is a side view of a seal module of a further example,
Figures 11A-C are cross-section views of the nasal bridge part the seal module of Figure 10 along line I-I of Figure 10 in different positions of adjustment of the nasal bridge part of the seal,
Figure 12 is a schematic side view of a respiratory interface of the invention comprising a nasal seal part and an oral seal part which are detachably magnetically coupled,
Figure 13 is a perspective view form above of an embodiment of an oral insert configured to be retained in a user's mouth when a mask is worn to assist in retaining the mask in position on the face of the user,
Figure 14A is longitudinal cross-section part view of a conduit connected to the end of an elbow of a mask by a magnetic coupling system according to an embodiment, and Figure 14B is longitudinal cross-section part view similar to Figure 14A but in which only a part of the conduit is shown in cross-section,
Figure 15 schematically shows an embodiment of headgear for a respiratory interface, comprising headgear parts and a magnetic coupling system arranged to magnetically couple headgear parts to one another,
Figures 16A and 16B show embodiments of headgear for a respiratory interface comprising a magnetic coupling system to magnetically hold headgear parts together,
Figure 17 shows an embodiment of headgear for a respiratory interface comprising a magnetic coupling system, and a decoupling tool,
Figure 18 is a schematic perspective view of a magnetic repulsing feature for a conduit,
Figure 19 is a schematic top view of the magnetic repulsing feature of figure 18,
Figure 20 is a schematic cross-sectional view of the magnetic repulsing feature of figure 18,
Figure 21 is a schematic detailed perspective view of the magnetic repulsing feature of figure 18,
Figure 22 is a schematic view of a respiratory interface comprising a conduit incorporating the magnetic repulsing feature of figure 18,
Figure 23 is a schematic perspective view of a magnetic coupling feature for a conduit,
Figure 24 is a schematic cross-sectional view of the magnetic coupling feature of figure 23,
Figure 25 is a schematic detail cross-sectional view of the magnetic coupling feature of figure 23,
Figure 26 is a schematic detailed perspective view of the magnetic coupling feature of figure 23,
Figure 27 is a schematic perspective view of headgear in an open configuration,
Figure 28a is a schematic cross-section view of the headgear strap taken through plane A-A of figure 25 and figure 28b is a schematic cross-section view of the headgear strap taken through plane B-B of figure 27,
Figure 29 is a schematic perspective view of the headgear of figure 25 in a wrapped configuration,
Figure 30 schematically shows another embodiment of headgear for a respiratory interface, comprising headgear parts and a magnetic coupling system arranged to magnetically couple headgear parts to one another,
Figure 31 is a schematic view of the headgear of figure 30 in a relatively large sized, in-use configuration,
Figure 32 is a schematic view of the headgear of figure 30 in a relatively small sized, in-use configuration,
Figure 33 schematically shows another embodiment of headgear for a respiratory interface, comprising headgear parts and a magnetic coupling system arranged to magnetically couple headgear parts to one another,
Figure 34 is a schematic view of the headgear of figure 33 in an open configuration,
Figure 35 is a schematic view of the headgear of figure 33 in a closed configuration,
Figure 36 is a schematic view of a seal module and elbow,
Figure 37 is a schematic cross-sectional view of the seal module and elbow of figure 36,
Figure 38a and 38b are schematic cross-sectional views of the seal module and elbow of figure 36, figure 38a shows the elbow in a misaligned position and figure 38b shows the elbow in an aligned position,
Figure 39 shows a rear view of a mask frame according to another embodiment,
Figure 40 shows a first seal module for use with the mask frame of figure 40,
Figure 41 shows a second seal module for use with the mask frame of figure 39,
Figures 42A to C show a sequence of interchanging first and second seal modules with a mask frame according to figure 39 and seal modules according to figures 40 and 41.

### DETAILED DESCRIPTION OF SPECIFIC EMBODIMENTS

Interfaces to which this disclosure relates can be used in the field of respiratory therapy. In some embodiments, the interfaces may have particular utility with forms of positive pressure respiratory therapy. For example, an interface can be used for administering continuous positive airway pressure ("CPAP") treatments, variable positive airway pressure ("VPAP") treatments and/or bi-level positive airway pressure ("BiPAP") treatments. The interface can be compatible with one or more different types of suitable CPAP systems.

### Magnetic coupling of seal module and mask frame

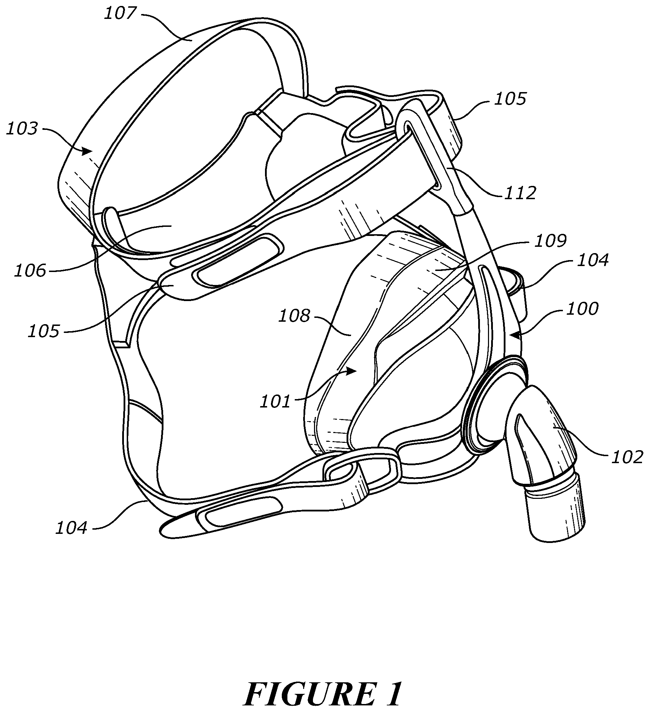

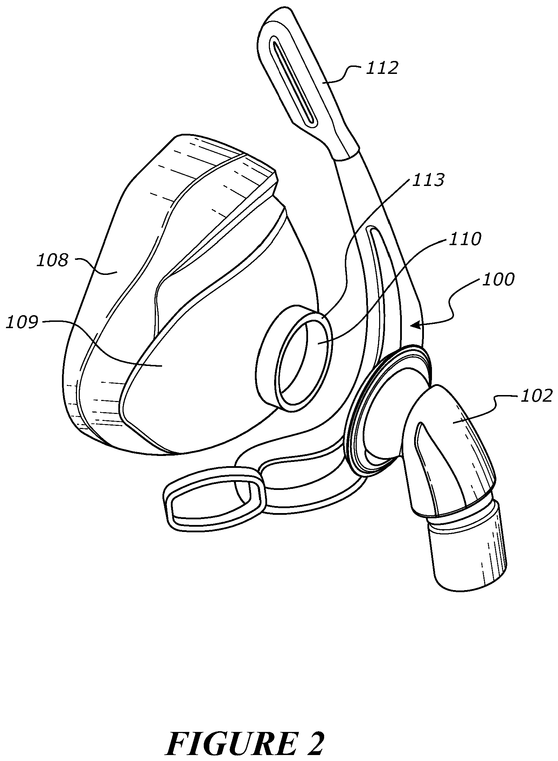

Figures 1 to 4 show a respiratory interface comprising frame 100 and seal module 101.The frame 100 can be rigid, substantially rigid or semi-rigid. For example, the frame can be at least partially made of a metal or rigid plastic, such as acrylic or polycarbonate or high-density polyethylene. The seal module 101 is a separate component from the frame 100 and couples to the user-facing side of the frame to assemble the interface for use, and is detachable from behind the frame for cleaning of the seal module for example. In the embodiment shown in Figures 1 and 2 the seal module covers a user's mouth and nose to deliver respiratory gases to the user. Alternatively the seal module may cover the nose only or mouth only, or comprise nasal nozzles or pillows or similar which enter into and seal against or within the nares of the wearer, or cannula which non-sealingly enter the nares. In further embodiments the seal module may cover the mouth and also comprise nozzles or pillows which enter the nares.

In the embodiment shown the interface comprises an elbow 102 by which an air delivery conduit couples to the interface, and in the embodiment shown the elbow couples to the frame 100 via a ball joint but alternatively the elbow may swivel about one axis only, or the air delivery conduit may couple directly to the interface without an elbow. The seal module 101 comprises an aperture into the seal module by which respiratory gases (hereinafter, air) enter the seal module from the elbow or conduit direct.

At least the part of the seal module 101 which contacts the user's face is formed of a soft material, such as a silicone material for example, to comfortably seal against the user's face. All or substantially all of the seal module may be formed from this material, which may be thicker towards and where the seal module interfaces with the frame to provide some structural support to the thinner face contacting part of the seal module, or alternatively as in the embodiment shown a forward part of seal module ie a frame interfacing part or shell part 109, may be formed of a rigid material such as acrylic or polycarbonate or high-density polyethylene, or a semi-rigid material (while seal part 108 which contacts the user's face is formed of the softer material). Alternatively again only a smaller part or parts of the seal module which connect the seal module to the frame may be formed of a rigid or semi-rigid material, such as a ring part around the air entry aperture into the seal module for example. Seal part 108 may be overmoulded, welded to shell part 109 or may attach to part 109 by permanent or detachable clipping including snap fitting, for example.

Embodiments of the interface may comprise one or more vent holes for expelling exhaled air. One or more vent holes may be provided in the seal module or elbow 102 for example.

Embodiments of the interface may comprise an anti-asphyxia valve, in an elbow for example or elsewhere on the interface. Headgear generally indicated at 103 holds the interface in position on the user's head when worn. In the embodiment shown the headgear comprises left and right lower straps 104 and left and right upper straps 105, which extend from a rear part 106 of the headgear, along the left and right sides of the user's head below and above the ears to couple to the frame 100 of the interface. The upper straps 105 couple to the top of a forehead support 112 of the frame 100, but in other embodiments may extend downwardly from above the ears, below the eyes, to connect to a frame not having a forehead support. Optionally the headgear may also comprise a top strap such as a crown strap 107 or forehead strap, and/or an occipital loop, and the headgear may be in various other forms. For example headgear may comprise only a rear strap and a crown or forehead strap, and a single strap along either side of the user's head or face to the mask. The rear part 106 of headgear can comprise a generally annular component comprising a back strap, a top strap and a pair of upright straps. The headgear may be formed from parts attached together, using for example ultrasonic welding, or may be a single unitary pieces (not formed from separate attached parts). The length of one or more of the headgear straps may be adjustable. Headgear is commonly formed at least in part from a soft flexible material such as a cloth covered foam material such as BREATHE-O-PRENE^{®} material for example, but may be formed from any other suitable material, such as in whole or part from a semi-rigid plastics material for example which may optionally be covered with a softer material. The headgear may be formed from parts attached together, using for example ultrasonic welding, or may be a single unitary piece (not formed from separate attached parts). The headgear may comprise one or more rounded edges, formed in any suitable manner, for example by applying heat and pressure to edges of the headgear. The headgear assembly can be configured to directly couple to the mask assembly without the use of clips. In some configurations, the pair of upper side straps and the pair of lower side straps can comprise ends with fasteners that loop through headgear attachments on the mask assembly and the fasteners can be configured to couple with complementary fasteners on the sides of the pair of upper side straps and the pair of lower side straps. Further disclosure regarding headgear is at the end of this description.

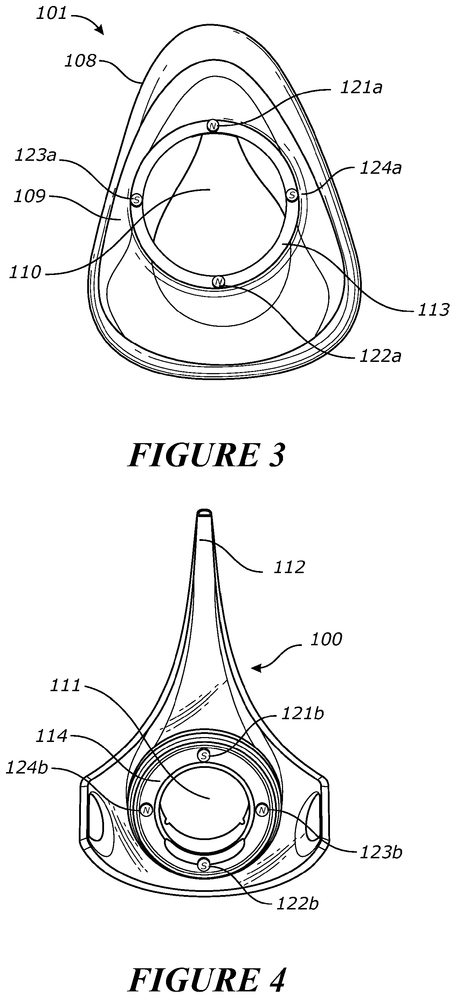

In the embodiment shown the seal module 101 couples to the mask frame 100 at or around aperture 110 in the front of the seal module through which air enters the seal module in use. When the seal module 101 is correctly fitted to the mask frame 100 seal module aperture 110 aligns with a corresponding aperture 111 through the mask frame from the elbow or conduit, through which air passes to the seal module. In the embodiment shown aperture 111 through the mask frame also defines the socket in the mask frame which receives the ball joint end of elbow 102. A magnetic coupling system couples the seal module 101 and mask frame 100, to hold the seal module on the mask frame. Figure 3 is a front view of the seal module and Figure 4 is a rear view of the mask frame ie shows the side of the mask frame which interfaces with the seal module. Magnets 121a, 122a, 123a and 124a are associated with rim portion 113 around of aperture 110 through the seal module. For example the magnets may be embedded in this rim portion during plastic moulding of the seal module. Magnets 121b, 122b, 123b and 124b are provided in rim portion 114 around the aperture through the mask frame, and again may for example be embedded in this rim portion 114 during plastic moulding.

The magnets in the seal module and frame are positioned so that when the seal module is positioned against the mask frame in the correct orientation of the seal module and mask frame relative to one another, magnetic attraction will hold the seal module on the mask frame. Thus each pair of magnets 121a-121b, 122a-122b, 123a-123b, and 124a-124b, are provided so that the opposite poles of each pair face one another when the seal module is correctly oriented relative to the mask frame, and then magnetic attraction holds the seal module and mask frame together. It is preferred to provide magnets in both the seal module and mask frame, but alternatively magnets may be provided in the seal module only and a ferrous component or components provided in the mask frame (about aperture 111), or vice versa, or some magnets may be provided in each of the seal module and mask frame and also ferrous components at other locations in each of the mask frame and seal module. At least one magnet or ferrous component is provided in the seal module and at least one ferrous component or magnet in the mask frame, or at least one magnet in each of the seal module and mask frame. In the embodiment shown four magnets are provided in each of the seal module and mask frame, approximately equi-distantly spaced about the apertures 110 and 111 as described.

In at least some embodiments at least two magnets are provided in the seal module 101 and at least two matching magnets in the mask frame 100, and the two magnets in the seal module have opposite poles facing the mask frame and the same for the two magnets in the mask frame facing the seal module. For example in the embodiment shown in Figures 3 and 4, magnets 121a and 122a in the seal module 101 have north poles facing the mask frame, and magnets 123a and 124a have south poles facing the mask frame. Magnets 121b and 122b in the mask frame 100 have south poles facing the seal module and magnets 123b and 124b in the mask frame have north poles facing the seal module. Thus in the correct orientation of the seal module and mask frame relative to one another all magnets attract, to hold the seal module on the mask frame, but if the seal module is offered to the mask frame in at least one or more incorrect orientations, the magnets repel. The magnets may be arranged so that a seal module will couple to the mask frame only in one (correct) orientation of the seal module relative to the mask frame. Such an orientation may be achieved by an asymmetrical arrangement of magnets upon the seal module, coupling to an identical asymmetrical arrangement of opposite pole magnets upon the mask frame.

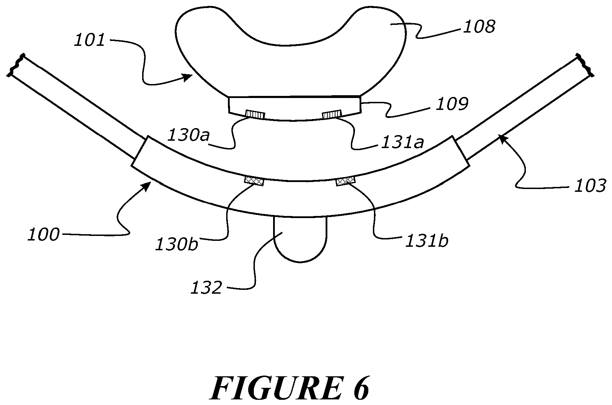

Figure 6 shows another embodiment of an interface, in which magnet 130a on a right hand side (as the interface is worn) of seal module 101 has its north pole facing mask frame 100 and magnet 131a on a left side of the seal module has its south pole facing the mask frame. Magnet 130b on right side of the mask frame 100 has its south pole facing the seal module 101 and magnet 131b on left side of the mask frame has its north pole facing the seal module 101. Two such magnets are provided in each of the seal module 101 and mask frame 102.The seal module 101 will only magnetically couple to the mask frame 100 when the seal module is offered to the mask frame 100 in the correct orientation, such that magnet 130a is opposite magnet 130b and magnet 131a opposite magnet 131b. In other orientations of the seal module relative to the mask frame, either the magnets will not align so that there will be no magnetic attraction of the seal module to the mask frame, or in a reversed (upside down) orientation of the seal module, magnets 131a and 130a in the seal module will face magnets 130b and 131b in the mask frame and the magnets will magnetically repel, so that the seal module and mask frame cannot be coupled together, making it apparent to the user that the orientation of the seal module relative to the mask frame is incorrect. Unless otherwise indicated in this and later figures similar reference numerals as in Figures 1 to 4 indicate similar components.

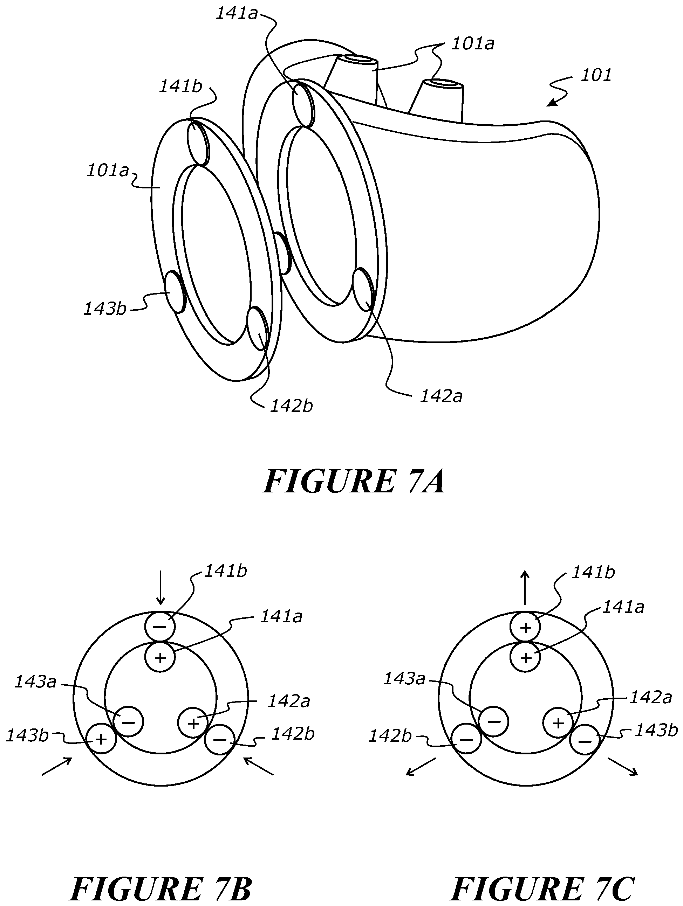

Figure 7A is a schematic view of a seal module 101 which comprises nozzles 101a and interfacing part of a mask frame 100 of an interface of another example, such as a frame rim 114, and Figures 7B and 7C are schematic views of magnet parts of the seal module and mask frame in correct and incorrect orientations relative to one another respectively. Three magnets 141a, 142a and 143a are provided in the seal module 101, and three magnets 141b, 142b and 143b in opposite locations in the mask frame. Magnets 141a and 142a in the seal module have similar poles facing the mask frame eg north poles. Magnet 143a in the seal module has an opposite pole facing the mask frame (opposite to magnets 141a and 142a) eg south pole. Similarly in the mask frame magnets 141b and 142b have similar poles facing the seal module eg south poles, and magnet 143b has an opposite pole facing the seal module eg north pole. Thus the seal module and mask frame will attract only in one (correct) orientation of the seal module relative to the mask frame, as illustrated by Figures 7B - correct orientation, and 7C - incorrect orientation.

Interfaces of the disclosure may rely solely on magnetic coupling of the seal module to the mask frame in use of the interface. Alternatively a magnetic coupling system may supplement, and be provided in addition, to mechanical coupling between the seal module and the mask frame, such as a friction fit, snap fit, or other interference fit, of formations on the seal module and mask frame to one another, either around the airway path through the mask frame into the seal module, or at other locations on the seal module and mask frame.

In some embodiments the interface may rely predominantly on mechanical coupling to hold the seal module on the mask frame, and a magnetic coupling system as described may be provided primarily to indicate correct and incorrect alignment of the seal module and mask frame when the user brings them together, so that coupling can only occur when the seal module and mask frame are offered to one another in correct alignment.

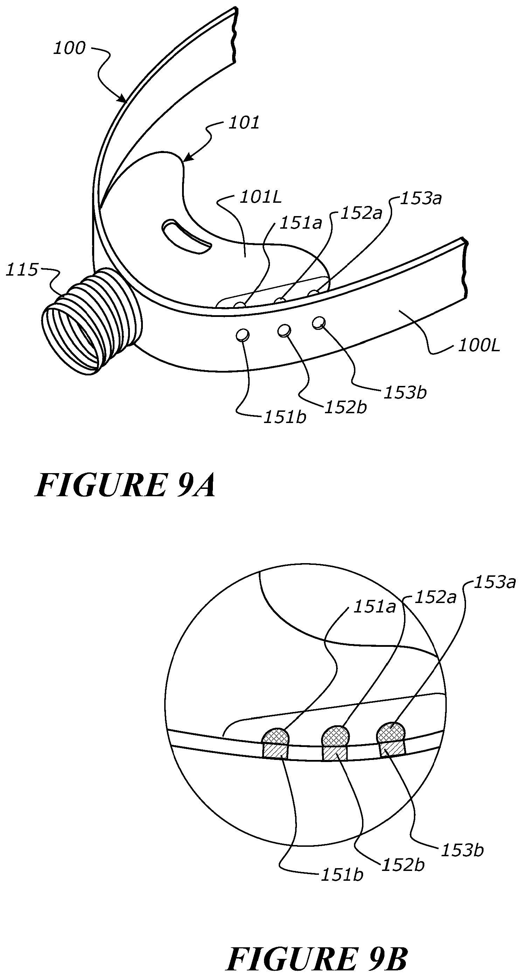

Figures 9A and 9B are schematic views of an embodiment of a respiratory interface in which magnets associated with the seal module and mask frame are provided not directly around the airway path, but at left and right sides of the seal module and mask frame. In this embodiment one or more magnets, such as magnets 151a, 152a, and 153a, are provided on the left side 101L of the seal module, which couple to one or more magnets (or ferrous parts), such as and magnets 151b, 152b and 153b, on the side of a left part 100L of the frame of the mask. Similar magnets are provided on the right side of the seal and corresponding right side of the frame (not shown). The magnets attract when the seal module and mask frame are correctly aligned relative to one another. Around the airway path connection ie the connection of an air entry aperture centrally positioned into the seal module 101, to an aperture through mask frame 100 from the conduit 115, formations may be provided for a mechanical connection between the seal module and mask frame, to hold or assist in holding the seal module to the mask frame, or alternatively coupling of the seal module to the mask frame may rely primarily on the magnetic coupling system and simply a gas seal may be provided between the seal module air gases entry aperture and the mask frame to prevent air leakage. In the embodiment shown the seal is an under-nose indirect seal but may be any other seal type as previously described.

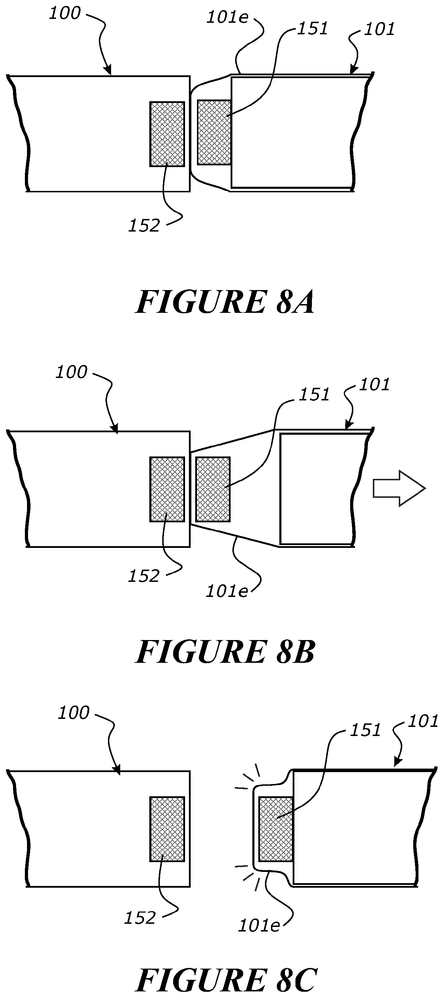

Figures 8A, 8B, and 8C are schematic cross-section views of interfacing parts of a seal module and mask frame of an interface of a further example. The objective of this embodiment is to produce an audible sound (eg 'click') on attachment and detachment of the seal module to/from one another. The interfacing parts of the seal module and mask frame are shown connected in Figure 8A, near connected in Figure 8B, and apart in Figure 8C. In this embodiment magnet or magnets 151 associated with the seal module 101 for example about the periphery of an air entry aperture into the seal module, are mounted to the seal module such that they can move relative to the balance of the seal module, in the direction of the arrow in Figure 8B between the magnet position shown in Figures 8A and 8C, and the magnet position shown in Figure 8B. For example the magnet(s) 151 may be embedded in a part 101e of the seal module designed to contact the mask frame 100, which part of the seal module is formed of a stretchable material. For example the seal module may be moulded from a soft synthetic elastic material; the magnets are embedded in the soft material; and the soft material around the magnets is elastically stretchable so that the magnets can move relative to the balance of the seal module. Alternatively the magnets may be captured on for example a rim of the seal module by an elastically stretchable fabric cover.

Figure 8A shows the seal module 101 connected or coupled to the mask frame. Figure 8C shows the seal module and mask frame separated. The normal position of the seal magnet(s) is as shown in Figure 8B. When the seal module is offered to the mask frame, as the seal module approaches the position shown in Figure 8C relative to the mask frame, and continues to approach the mask frame, magnetic attraction will pull the magnet(s) of the seal module towards the magnet(s) 152 of the mask frame, stretching the magnet(s) 151 of the seal module away from the balance of the seal module as shown in Figure 8B, until they contact the mask frame (then pulling the seal module fully towards the mask frame to the fully coupled position shown in Figure 8A). As the seal module is brought towards the frame, at a break point at which the magnetic attraction of the seal and frame magnets to one another exceeds the elastic retractive force of the elastic part or stretchable fabric cover 101e of the seal module, the seal magnet(s) 151 will snap from the position of Figure 8C to that of Figure 8B and the impact of the seal module magnet(s) against the frame or frame magnets may produce an audible click. Similarly when the seal module is pulled from the mask frame ie the seal module is moved from the position shown in Figure 8B to that shown in Figure 8C, the magnet(s) 151 of the seal module will come away from the mask frame and snap back into position as shown in Figure 8C which may again producing an audible click. Alternatively the magnet(s) (or ferrous part(s)) on the frame may be elastically movably mounted, or the magnets on both the seal module and frame. It may be advantageous that the magnet(s) movement as described produces an audible sound on coupling and uncoupling as an indicator to the user but in other embodiments the same structures as described may be employed but designed to couple and uncouple silently.

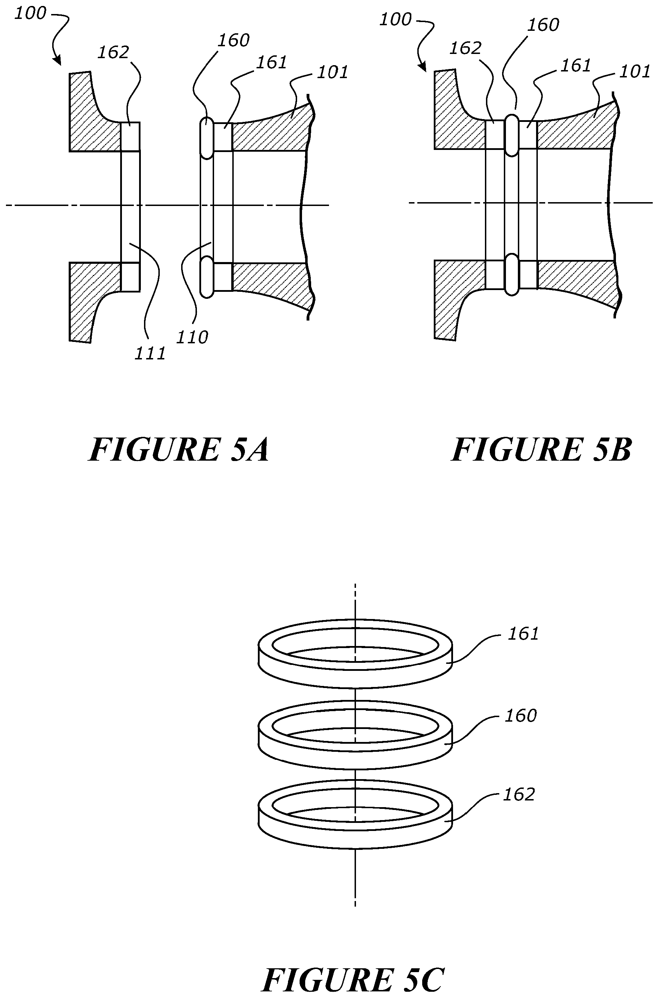

Figures 5A and 5B are schematic cross-section views of interfacing parts of a seal module and mask frame of an interface of another embodiment, separated in Figure 5A and connected in Figure 5B, and Figure 5C is a schematic exploded view of magnet parts of the seal module and mask frame and a gel pad between as will be further described. This embodiment is similar to others previously described in which magnets or magnets and one or more ferrous components are provided on a seal module 101 and mask frame 100 about the airway path through the interface. In this embodiment a ring 160 of a compressible material is provided on the seal module 101 forward of the magnet(s) 161 about the seal module gases entry aperture 110 as shown. The compressible ring 160 may for example comprise a ring of captured gel ie gel captured in an annular membrane, or a compressible synthetic material or rubber ring for example. Figure 5A shows the seal module 101 and mask frame 100 separated and Figure 5B shows the seal module and mask frame coupled. When the seal module and mask frame are coupled the compressible ring 160 is between the magnet(s) 161 and 162 on the seal module and the mask frame, and may be compressed slightly. This may assist in ensuring an airtight seal between the seal module and mask frame for example.

In the embodiment shown a single magnet is provided on each of the seal module and mask frame, each in the form of a ring magnet as shown in Figure 5C. Alternatively the magnets 161 and 162 on one or both of the seal module and mask frame may comprise two or more curved segments which collectively define a ring around the airway path through each of the seal module and mask frame, or alternatively again the magnets may be multiple smaller individual magnets as shown for other embodiments.

In each case and in any embodiment of the disclosure, a magnet or magnets may comprise a segment of solid (non-particulate) magnet material, or alternatively a particulate magnetic material ie particles of a magnetic material embedded by plastic moulding for example, for example in the seal module or mask frame. Particulate material may form a ring magnet as in Figure 5 or multiple individual magnets as in other embodiments.

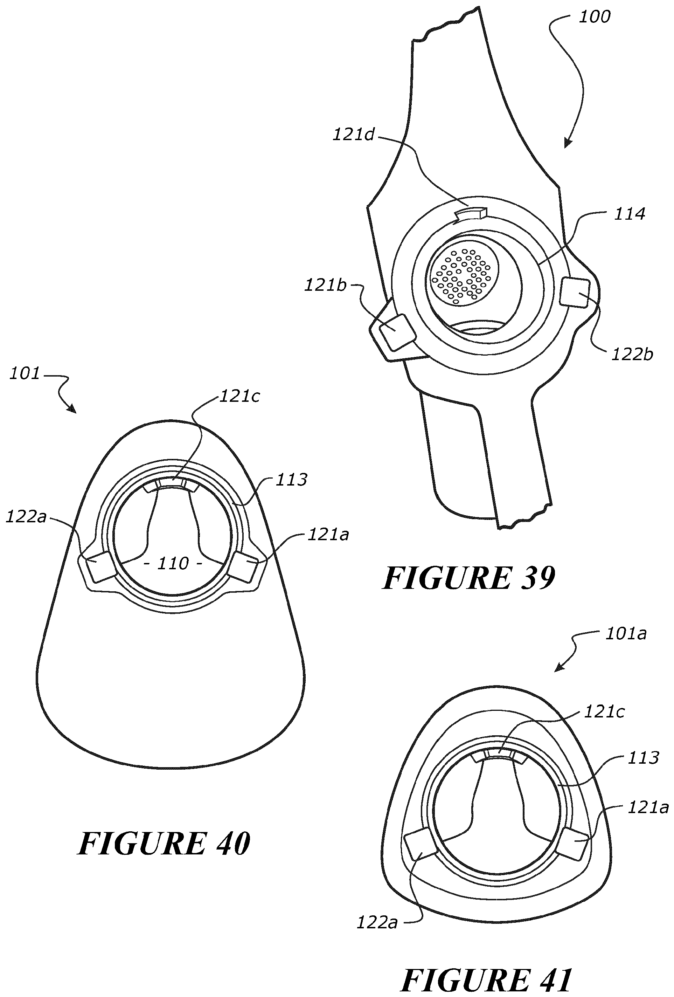

Figures 39 -42 show a further embodiment of seal module to mask frame magnetic coupling that allows a method of replacing or switching seal modules without removal of headgear from a user.

Figure 39 is a rear view of a mask frame 100 i.e. shows the side of the mask frame which interfaces with the seal module, comparable to Figure 4; meanwhile Figure 40 is a front view of a seal module 101 for covering the nose and mouth of a user, comparable to Figure 3. Magnets 121a and 122a are set into recesses on the seal module 101. The magnets 121a and 122a are located on opposite sides of an aperture 110 through the seal module 101. Surfaces of the magnets are flush with an outer surface of the seal module. This outer surface is a surface of radial flanges associated with a rim portion 113 of the seal module around the aperture 110. In the illustrated form magnets 121a and 122a are arranged opposite each other at a lower portion of the rim portion 113. The positions of the magnets 121a and 122a may be described as being at approximately four and eight o'clock positions respectively. The seal module also comprises a locator 121c in an upper portion. The locator is in an uppermost position upon the seal module 101. The position of the locator 121c may be described as being at approximately a 12 o'clock position. The magnets 121a and 122a are equidistantly spaced from the locator 121c.

Corresponding magnets 121b and 122b are likewise set into recesses in the mask frame 100. The magnets are located on opposite sides of an aperture through the mask frame. The magnets 121b and 122b on the mask frame are located so that they will be opposed to and engaged with respective magnets 121a and 122b on the seal module 101 when the seal module is joined to the mask frame. Surfaces of the magnets are flush with an inner surface of the mask frame. This inner surface is a surface of radial flanges associated with a rim portion 114 of the mask frame around the aperture. In the illustrated form magnets 121b and 122b are arranged opposite each other at a lower portion of the rim portion 114. The positions of the magnets 121b and 122b may be described as being at approximately four and eight o'clock positions respectively. The mask frame also comprises a locator 121d in an upper portion. The locator is in an uppermost position upon the mask frame. The position of the locator 121d may be described as being at approximately a 12 o'clock position. The magnets 121b and 122b are equidistantly spaced from the locator 121d.

Although the illustrated embodiment shows the seal module and mask frame having two magnets and a single locator, it is to be appreciated that in other embodiments, the seal module and mask frame could each have 3, 4, 5, 6, 7, 8 or more magnets and/or 2, 3, 4, 5, 6, 7, 8 or more locators.

Locating features 121c and 121d may have any suitable mating profiles that enable the locating features to inter-engage with each other. The profiles for the locating features may be shaped with keys that limit fitment between the locating features to particular orientations.. The locator 121c on the seal module is a projection (male part) that projects forwardly of the seal module. The locator 121d on the mask frame is a recess (female part) that extends into the mask frame. The recess locator 121d is configured to receive the projection locator 121c. It is to be appreciated that in other embodiments, the locator on the seal module may be a recess and the locator on the mask frame may be a projection. The locator projection 121c has a wedge shape. That is, the locator projection 121c tapers as it extends away from the seal module. Such a wedge shape enables guidance of the locator projection into the locator recess and subsequent engagement of the magnets even when the seal module and mask frame are not perfectly aligned.

When the seal module is coupled to the mask frame, the receipt of the projection locator 121c in the recess locator 121d provides resistance to rotation of the seal module relative to the mask frame about the seal module aperture. However, the inter-engagement of the locators provides no limitation to linear movement of the seal module away from the mask frame. The engagement of the respective magnets 121a,b and 122a,b on the seal module and the mask frame, provides resistance to the linear movement of the seal module away from the mask frame but is weak at resisting rotation of the seal module relative to the mask frame about the seal module aperture. Accordingly, the locators and magnets provide a combination of mechanical and magnetic engagements to hold the seal module to the mask frame. The locators and magnets provide threshold resistance to disengagement of the seal module from the mask frame under rotation or linear forces. The threshold rotation force in a direction about the seal module aperture to cause disengagement is higher than the threshold linear force.

Figure 41 shows a nasal seal module 101a that otherwise has the same configuration of magnets 121a, 122a and locating feature 121c about a rim portion 113 as the full nose/mouth seal module 101 of Figure 40. In this way it is compatible to be coupled with the rear of the same mask frame 100 shown in Figure 39.

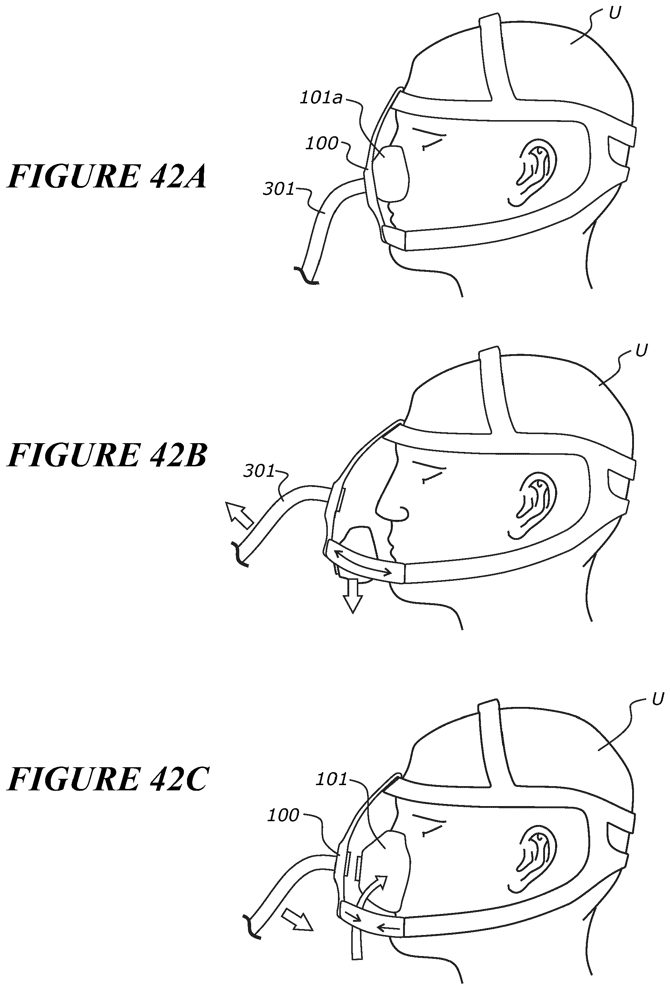

Figures 42A-C illustrate a method that can be implemented using the mask frame 100 and seal module components 101 and 101a and, particularly, a method of swapping seal module components without removal of the headgear.

A user U is shown in Figure 42A wearing headgear and a nasal seal module 101a in place upon mask frame 100. Headgear can be exemplified according to principles disclosed in the Applicant's PCT Publication No. WO2014/175752, e.g. paragraphs [0145] - [0149], [0159] - [0163], wherein with respect to patient interfaces that seal on the face of the user, the interface (e.g., mask) in cooperation with the user's face creates a sealed chamber. Pressurized breathing gases are delivered to the sealed chamber, which generates a force tending to move the mask away from the user's face. This force is generally equal to the (projected) seal area multiplied by the positive pressure and is often referred to as the "blow-off force. A function of the headgear is to restrain the mask in response to the blow-off force to keep the mask in equilibrium sealed against the face of the user. The blow-off force stresses the headgear in an attempt to elongate it, which places the headgear under tension.

In addition, the headgear applies a force to the user's head over an area with which the headgear is in contact. The force applied to the contact area can be referred to as the "skin pressure" of the headgear. As the air pressure within the chamber defined by the seal or the combination of the seal and the frame increases, the force applied by the headgear attempts to restrain the interface assembly from lifting from the face. As such, the force applied by the headgear generally will increase to oppose the increasing force resulting from the increasing pressure within the mask. The blow-off forces will vary for different types and sizes of interfaces at any given pressure. Nevertheless, at lower pressures, or no pressure in the case of cannulas, less force is required to oppose the blow-off forces.

Accordingly, headgear assemblies preferably can be designed to achieve a "balanced fit." In some configurations, the headgear assemblies generally comprise a stretch component (also referred to as elastic), a non-stretch component (also referred to as inelastic or non-elastic), a mechanism that restricts extension of the headgear, and a coupling that can join the headgear assembly to the mounting points, for example but without limitation. In at least some configurations, the balanced fit can be achieved by creating a substantially non-stretch path to resolve the stresses in the headgear when in use or in response to normal operational forces (e.g., blow-off and/or hose pulls forces, plus a reserve, if desirable). At higher forces than seen in use, the headgear can exhibit stretch-like behaviour for donning. In some configurations, the headgear assembly may not include a stretch component. For example, the headgear could be manually extended and retracted.

The stretch components, when present, can have any suitable configuration. The stretch components can be any component that has a tensile modulus of less than about 30 MPa. The tensile modulus is the mathematical description of the tendency to be deformed elastically (i.e., non-permanently) along an axis when forces are applied along that axis; tensile modulus is the ratio of stress to corresponding strain when a material behaves elastically. In some configurations, the stretch component can be a coated, spun yarn material and the stretch component can include materials such as, but not limited to, rubber and spandex or elastane (e.g., LYCRA). In some configurations, the stretch component can be a strap or a combination of straps. In some configurations, the stretch component can be formed of a stretchable or elastic material. In some configurations, the stretch component enables the headgear to be expanded or lengthened and the stretch component also provides a retraction force that serves to contract or shorten the headgear. The contraction, or shortening, can occur as a result of the elastic properties of the stretch component. The contraction, or shortening, allows the headgear to more closely match the user's head circumference (plus the size of the mask). Generally, the headgear length is defined by a relaxed length and the headgear seeks to return to that length and it is this returning toward the relaxed length after elongation that is meant by contraction unless otherwise apparent.

The non-stretch components can serve as a stretch limiter. The non-stretch components can have any suitable configuration. In some configurations, the non-stretch components have a higher modulus of elasticity compared to the stretch components. The stretch components can be any component that has a tensile modulus of more than about 30 MPa. In some configurations, the non-stretch components restrict elongation of the headgear due to forces that are lower than a specified yield force. In some configurations, the yield point of the non-stretch material is higher than any anticipated loading to be applied to the headgear. In some configurations, the non-stretch components resist elongation of the headgear once the headgear has been fitted to the head. In some configurations, the non- stretch components resist elongation of the headgear once the headgear has been fitted to the head and the CPAP pressure has been applied to the mask. Thus, in some configurations, the non-stretch components (in some cases, in combination with the mechanisms discussed below) can thwart or resist elongation of the stretch components at least when CPAP pressure is applied. In the case of a cannula, the non-stretch components can resist the movement of the cannula under the influence of external forces, such as hose pull.

The mechanism can be any suitable mechanism that can limit expansion or elongation of the headgear when a force lower than a specified yield force is applied to the headgear. In some configurations, the mechanism operates without an effort by the user (e.g., the mechanism is automatic). That is, in at least some configurations, the mechanism can automatically move or switch to a mode in which extension or expansion is limited below the specified yield force. However, effort may be required for the user to don the mask, such as effort above the yield force to extend the headgear. In some configurations, the mechanism can apply a motion resistance force that can limit the extension or expansion of the headgear when a force lower than the specified yield force is applied to the headgear. In some such configurations, the motion resistance force can be a friction force. The specified yield force, that is, the force at which the headgear mechanism's motion resistance forces are overcome and elongation of the headgear becomes possible, may be determined by (1) the maximum blow-off force that is possible for the specific mask in use when a range of about 4-20 cmH20 pressure is anticipated and (2) a reserve to allow for any pulling of the CPAP hose and differences in user fit preferences. The reserve, generally defined as the difference between the lengthening or extension force and the maximum balanced fit force, can provide a buffer above the balance fit force, in which additional forces can be applied to the headgear without substantial elongation of the headgear occurring. The reserve force component can compensate for any additional force, such as hose pull, that may act to pull the headgear from the user's head. In some configurations, the motion resistance force can be applied to restrict extension of the headgear while releasing to allow retraction or contraction of the headgear. In some configurations, the mechanism can use one-way friction to lock or otherwise secure the headgear length. For example, the length can be secured using a factional force that can only be overcome by a force that exceeds the blow-off force with minimal extension. Such mechanisms can be referred to herein as a directional locking arrangement or directional lock. The term "lock" as used herein is intended to cover mechanisms that secure the headgear length in response to certain forces, such as blow-off forces and/or hose pull forces. A "lock" does not necessarily secure the headgear length in response to all forces. Preferably, in some configurations, the retention force of the lock ("lock force") can be overcome, such as by manually-applied forces during the application portion of the fitment process.

To remove the interface while wearing the headgear described below, the seal can be pulled forward with a force greater than the mechanism's maximum holding force. This causes the headgear to lengthen and which enables the seal to be pulled away from the face and over the user's head. Once removed, the lack of forces on the headgear will cause the headgear to automatically retract to its relaxed size.

In some configurations, the headgear applies a three phase force extension fit profile. In the application phase, the headgear is stretched to go over the head of a user. A load curve features a steep rise in load for the initial extension of the headgear that then transitions to a generally constant, flat extension curve as the headgear is further stretched to accommodate larger head circumferences. In the adjustment phase, the headgear retracts and returns from a stretched condition until a desired fit is achieved. The load curve shows an initial decrease in load as the headgear retracts to fit onto the user's head and also illustrates a low load force as the headgear further retracts to fit the user's head circumference. In the third phase, the balanced fit phase, the headgear adjusts to hold its position on the user's head as CPAP pressure is applied. The load curve illustrates that a rise in load force of the headgear balances with the blow-off force due to the CPAP pressure and also resists additional forces, such as hose pull. In the case of a cannula embodiment, a balanced fit can be achieved at the end of phase two, and phase three typically will only be initiated if and when an external force, such as hose pull, is experienced.

As discussed above, the load curve will have a steep rise because the headgear has initial resistance to stretch as it is stretched to accommodate the user's head. The initial resistance can relate to overcoming the resistance that will resist elongation. Once the load has reached a yield force of the mechanism of the headgear, the load curve transitions to a substantially flat, generally constant extension curve as the headgear stretches further with little increase in load force for greater amounts of headgear extension.

In the second phase, the headgear has been sufficiently stretched or elongated to fit over the user's head and the headgear has been released into position. Once a desired positioning has been achieved, the headgear returns from the stretched condition (e.g., over-elongated position) and the load force sharply declines. After this reduction in force due to retraction of the headgear to fit the user's head, the load curve remains low 226 as the headgear remains fitted to the user's head. The headgear that typifies many features, aspects and advantages of the present disclosure features a first high load required to cause elongation and a second lower load at which the headgear contracts. In other words, the headgear contracts at a lower load than required to cause elongation and a hysteresis is the provided effect. In some configurations, the headgear has a delay in length change while the force changes dramatically when changing from an elongation mode to a contraction mode. In some configurations, the change in length of the interface circumference (including the headgear assembly) lags behind changes in load (i.e., force) when the interface length changes from elongation to contraction. Moreover, in some configurations, during elongation, as the force increases, the length increases more than the decrease in length during the decrease in force.

A balanced fit is achieved in the balanced fit phase, in which the force of the headgear balances the blow-off force of the CPAP pressure. As mentioned above, the headgear adjusts to hold its length as CPAP pressure is applied. The load curve will show the rise in the load force that balances the blow-off force. The balanced fit produces a higher load than the retraction force of the headgear. The balanced fit component is the increasing force in the strap of the headgear that provides an equal and opposite force to the blow-off force. However, this force is also lower than the lengthening or extension curve. In some configurations, the slope of the balanced fit section is related to, influenced by, or can be substantially the same as the rise and/or the decline in the load force during retraction of the headgear. In some configurations, the slope of the balanced fit section is steeper than the slope in the decline in the load force. In some configurations, the slope of the balanced fit section is greater than the slope in the initial rise during lengthening of the headgear.

Figure 42B shows the process of removing nasal seal module 101a, ie, by gripping a lower part of mask frame 100 and pulling it away from the face of user U, while it remains strapped to the head, to naturally form a gap G, against the bias of an elastic portion of a headgear strap and by decoupling of the magnetic connection of the type described herein.

Still with reference to Figure 42B the seal module 101a is then gripped at its accessible, bottommost, end and pulled toward the face of user U, thereby breaking the engagement between magnets 121a/121b and 122a/122b respectively. Nasal seal module 101a can then be decoupled from the posterior of the frame and removed through gap G between the user's face and mask frame 100. The force applied by the user U to achieve disengagement has to overcome the threshold forces that resist such disengagement. Disengagement can be achieved by 'peeling' the seal module away from the mask frame. By this process, the locator projection acts as a pivot whereby the seal module is pivoted upwardly about the locator projection to break the engagement between the magnets. Pivoting about the locator projection is facilitated by the wedge shape of the projection and that the height of the projection is less than the height if the locator recess.

With gap G still open a replacement seal module 101 can be inserted into the space in front of the face and connected to the rear side of the frame. The replacement seal module 101 may be the same type of seal module or a different type. For example, the replacement module may be a full face seal module where the original seal module is a nasal seal module. Mask frame 100 is then released to close gap G so that the replacement seal is brought into engagement with the user's face. Magnetic connections 121a/121b and 122a/122b respectively join frame 100 to the 'replacement' seal module 101, aided by locating features 121c/121d to correctly orient the components and the magnets themselves which will tend to pull the assembly into place by magnetic forces. In particular, wedge shaped locator 121c approaches locator recess 121d to guide mask frame 100 into position against seal module 101 before or at the same time as the corresponding magnets begin to engage by magnetic attraction.

In the illustrated form magnetic connections 121a to 121b etc. allows for the seal module 101/101a to be quickly and easily swapped out without the need to completely remove the full headgear or operate any complicated clipping means. Magnets being located at the lower part of the aperture with a locating feature at the top enables a seal module to be 'peeled' away by a relatively small force. Furthermore, magnets pull the parts into correct alignment, such that initial alignment does not need to be perfect between the rear/posterior of the mask frame and front/anterior of the seal module. The magnetic attachment system as described allows seal modules to be interchanged in the dark; e.g. where a user may want to change between a full face and nasal mask during the night for comfort. It is intended that the conduit aperture is located in such a place that when the mask frame 100 is connected to either the nasal or full face module, the apex/bridge of the seal module will be in the same place relative to a user's facial geometry.

### Magnetic hold of adjustment of seal

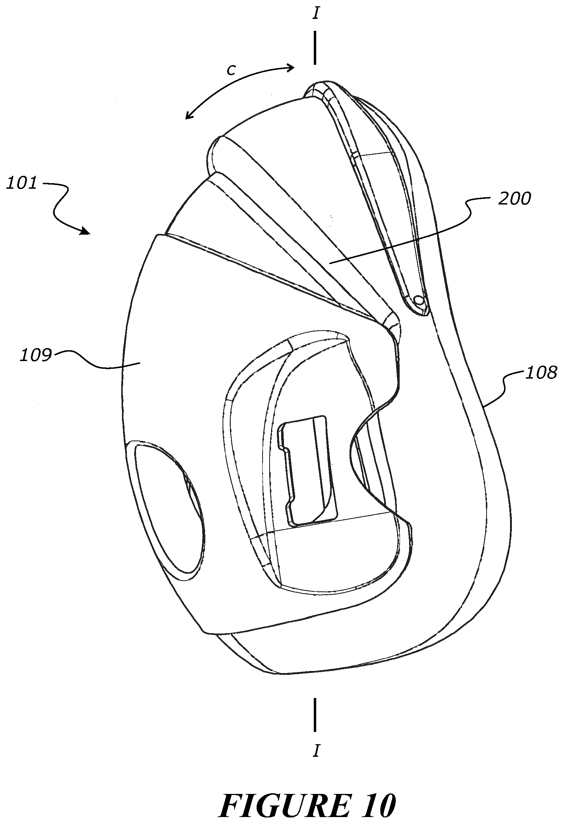

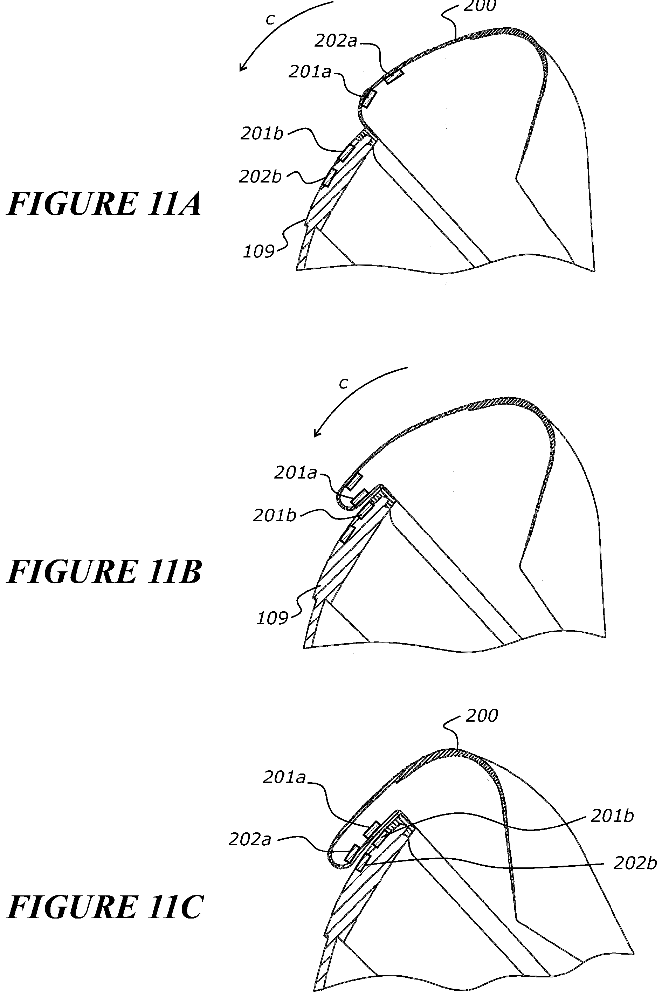

Figures 10 and 11A-C show, and illustrate the operation of, a seal comprising at least one magnet or ferrous part associated with the seal to hold a first part of the seal in position relative to another part of the seal or part of the mask frame of the respiratory interface, to magnetically hold an adjustment position of a part of the seal. In the embodiment shown multiple magnets and/or ferrous parts are provided in a nasal bridge part 200 of the seal, to hold in the nasal bridge part of the seal in a selected adjustment position. The nasal bridge part 200 of the seal is compliant as indicated by arrow C in Figures 10, 11A and 11B, enabling the nasal bridge part of the soft flexible seal to move forward of its normal position shown in Figure 11A, to for example either of the two positions shown in Figures 11B and 11C, to accommodate users with different depths of nasal bridge, so that the seal does not place excessive pressure on the nasal bridge area of the nose of the wearer. Such a compliant nasal bridge is described in our international patent publication WO2012/140514.

Magnets 201a and 202a are embedded in a forward part of the soft moulded material of the nasal bridge portion of the seal which moves to provide nasal bridge compliance of the seal, and magnets 201b and 202b are embedded in an adjacent (non-moving) part of the seal for example a shell part of the seal module formed of a relatively harder material (as escribed in relation to Figures 1 to 4 for example).The magnets particularly in the thin wall section flexing or folding nasal bridge part of the seal may comprise zones of embedded particulate material moulded in the silicone or other soft material from which the seal is formed, which extend transversely across the nasal bridge part of the seal from left to right for example (magnetic lines).The magnets are arranged with their poles oriented such that magnets will attract in defined discrete positions of adjustment. Two such positions are shown in Figures 11B and 11C. If the nasal bridge part of the seal is moved from the 'normal' position of Figure 11A, to the position of Figure 11B, to accommodate a medium depth nasal bridge user, in the position of Figure 11B magnets 201a embedded in the nasal bridge portion of the seal and 201b embedded in the non-moving part of the seal, will align to hold the seal in this position of adjustment. However if the user pushes the nasal bridge part of the seal further forward, to the position shown in Figure 11C, to accommodate a higher depth nasal bridge, a different pair of magnets namely magnets 202a embedded in the nasal bridge portion of the seal and 202b embedded in the non-moving part of the seal, will align also as shown, or instead, to hold this adjustment position.

The embodiment shown has two positions of magnetic hold of adjustment but in other embodiments magnets may be provided in the seal and/or frame to provide three or more, on only one, magnetic adjustment hold position.

In the embodiment shown the compliant nasal bridge is provided in the seal module of an interface comprising a mask frame and separate seal module but such a magnetic adjustment system of the nasal bridge may be provided in the compliant nasal bridge of a seal which is non-detachable from the mask frame for example a seal which is permanently overmoulded to the mask frame.

### Nasal to full face magnetically connected seal

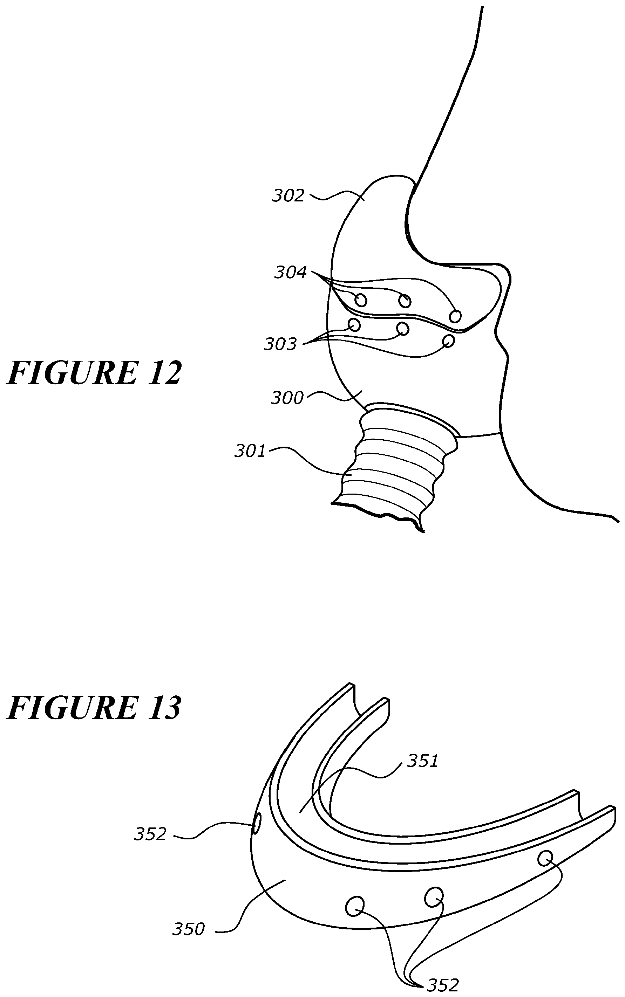

Figure 12 is a schematic side view of a respiratory interface of the invention comprising a nasal seal part and an oral seal part which are detachably magnetically coupled. The interface comprises an oral seal first part 300 to deliver gases through the mouth, supplied in the embodiment shown to the interface via conduit 301, and a nasal seal second part 302 to deliver gases to the nose also. The nasal seal part 301 may be an indirect or direct nasal seal as described. A magnetic coupling system magnetically couples the oral seal part 300 and nasal seal part 302 together to form, when coupled, a combined oro-nasal seal.

In the embodiment shown the oral seal part 300 comprises magnets 303 around an upper peripheral part and the nasal seal part comprises magnets 304 around a lower peripheral part, both on left and right sides and across the front of the seal, or alternatively a single strip magnet in each seal part instead of discrete magnets as shown, or embedded particulate magnetic material, embedded in the seal during moulding for example, which magnetically couple the seal parts together or embedded particulate magnetic material, embedded in a soft synthetic insert 350 during plastic moulding for example.

The seal parts may be attached to one another or coupled, and detached from one another, so that the interface may be used with both seal pats together or with the oral seal part only. In an alternative embodiment the conduit may connect to a nasal seal part so that an oral seal part may be magnetically coupled to or detached from the nasal seal part enabling the nasal seal part to be used alone.

In the embodiment shown the two seal parts 300 and 302 magnetically couple together but in an alternative embodiment one or both of the seal parts may also or alternatively magnetically couple to a common mask frame, again so that one or other of the seal parts may be detached to enable the interface to be used as an oral-only, or nasal-only, interface if the other seal part is detached.

In some embodiments the magnetic coupling system comprises at least one magnet associated with one of the first and second seal parts or mask frame and at least one ferrous part associated with one of the first and second seal parts or mask frame. In other embodiments the magnetic coupling system comprises magnets associated with each of the first and second seal parts or one of the first and second seal parts and the mask frame.
In some embodiments at least one magnet associated with one of the first or second seal parts or the mask frame and at least one magnet associated with another of the first or second seal parts or the mask frame are provided to the seal parts or mask frame such that they attract when the seal parts are coupled each other or to the mask frame in a predetermined correct orientation and repel in any orientation other than said predetermined correct orientation.

### Magnetic mouth connection

Figure 13 is a perspective view from above of an embodiment of an oral insert configured to be retained in a user's mouth when a mask is worn, to assist in retaining the mask in position on the face of the user. Oral insert 350 is configured to be retained at least in part on the teeth of the user. In the embodiment shown the oral insert 350 has an approximate U-shape as shown, and comprises an upwardly facing open channel 351, so that it is configured to be retained on the left right and front upper teeth of the user when worn. When worn the oral insert 350 remains attached to the teeth securely, but is shaped and sized so as to be as minimally invasive and as maximally comfortable as possible. The insert may be formed of a material which is soft but has some structural rigidity, such as a silicone material for example. The dimensions across the upwardly facing channel 351 are such that the insert will remain wedged on the teeth.

The insert comprises magnets 352 spaced around its forward periphery as shown, or alternatively a single magnetic strip may extend around the front periphery of the insert. The magnets or magnetic strip may comprise a solid magnet or magnets or embedded particulate magnetic material, embedded in a soft synthetic insert 350 during plastic moulding for example.

A corresponding magnet or magnets are provided on a mask (not shown in Figure 13) particularly the seal or cushion at the left and right sides of in the upper lip region or across the upper lip region, so that when the oral insert 350 is in place on the user's teeth, and the mask is placed in the correct location on the face, the mask will magnetically couple to the oral insert 350 to at least assist in retaining the mask in position on the face of the user. This may be in addition to headgear coupled to the mask, so that the magnetic system stabilises the mask on the face for example, or in some embodiments the interface does not also comprise headgear and the magnetic coupling system through the oral insert 350 solely retains the mask in position when worn by the user.

In embodiments the magnetic coupling system comprises at least one magnet associated with one of the mask or oral insert and at least one ferrous part associated with the other of the mask or oral insert. In embodiments the magnetic coupling system comprises magnets associated with both of the mask or oral insert.

### Magnetic swivel

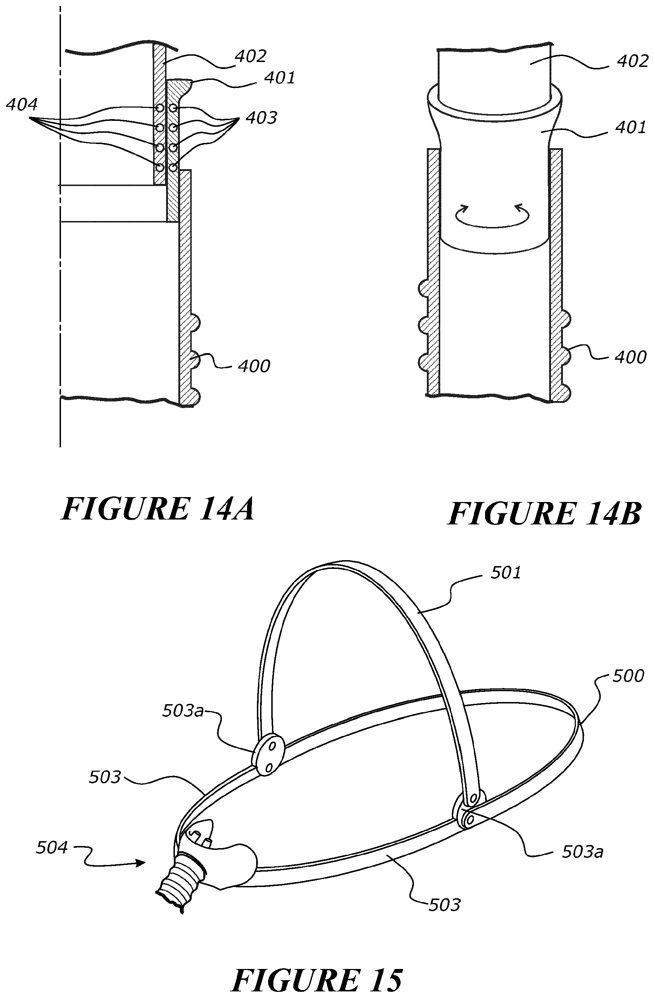

Figure 14A is a longitudinal cross-section part view of a conduit connected to the end of an elbow of a mask by a magnetic coupling system according to an embodiment, and Figure 14B is a longitudinal cross-section part view similar to Figure 14A but in which only a part of the conduit is shown in cross-section. In these figures flexible conduit 400, for example from a CPAP machine to a respiratory interface, comprises at one end a rigid or semi-rigid annular part or fitting 401, which is received over a rigid or semi-rigid annular fitting 402 which is part of or attached to the interface. For example part 402 may be the lower end of an elbow connecting to the mask, or may be a fitting on the bottom end of a short lead in tube forming part of the mask. Annular magnets 403 are embedded in the part 401, for example during plastic moulding of the part 401, and corresponding annular magnets 404 are embedded in the part 402 during moulding of this part for example. There may be one or more magnets in each part, which may be discrete ring or segmented ring or magnets or embedded particulate magnetic material magnets. The magnets 403 and 404 are positioned, and part 401 has a slight internal taper and part 402 a slight external taper, so that when the parts 401 and 402 are brought together the magnets will align and magnetically couple part 401 on the end of conduit 400 to the part 402 on the bottom end of the elbow or mask lead in tube. The magnetic coupling may or may not form a frictionless or near frictionless swivel ie the part 401 can rotate about the longitudinal axis of the coupling without becoming detached from the part 402.

In the embodiment shown the magnet coupling system is between conduit 400 and part 402 which is described above as at the bottom end of an elbow or lead in tube to a mask but alternatively part 402 may be comprising a fitting such as a fitting of a CPAP machine for coupling a conduit to a CPAP machine or in any other context, or may be provided at the end of another length of conduit, for coupling together two conduit lengths, for example.

In embodiments the magnetic coupling system comprises at least one magnet associated with one of the conduit to the mask and the mask or a lead in tube to the mask and at least one ferrous part associated with another of the conduit to the mask and the mask or a lead in tube to the mask. In embodiments the magnetic coupling system comprises magnets associated with both of the conduit to the mask and the mask or a lead in tube to the mask.

In embodiments at least one magnet associated with one of the conduit to the mask and the mask or a lead in tube to the mask and at least one magnet associated with another of the conduit to the mask and the mask or a lead in tube to the mask are provided to the conduit and the mask or a lead in tube to the mask such that they attract when the conduit and the mask or a lead in tube to the mask are coupled each other or to the mask frame in a predetermined correct orientation and repel in any orientation other than said predetermined correct orientation.

### Self-centring

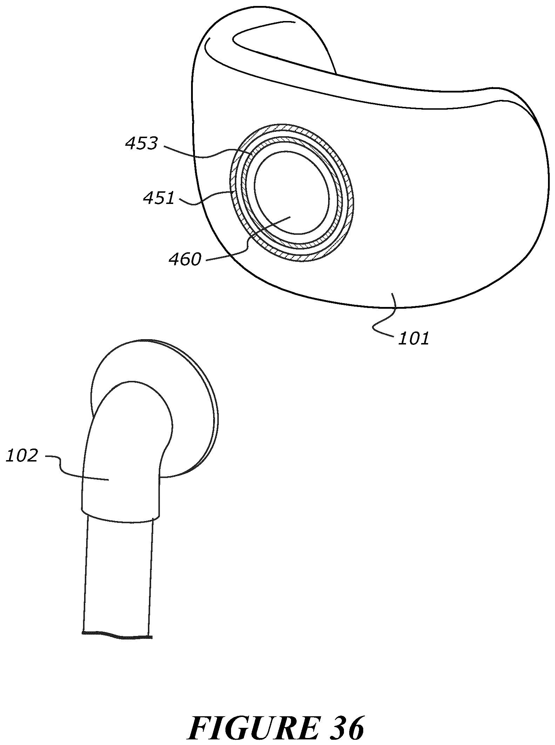

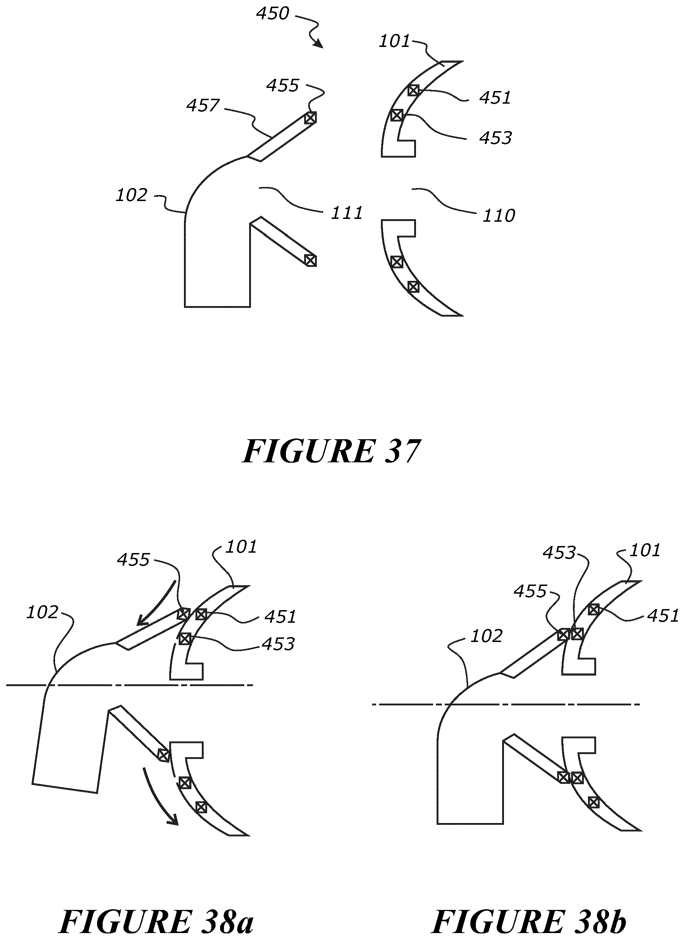

Figures 36 and 37 schematically show components of an embodiment of a respiratory interface having a magnetic coupling system 450 for connecting a conduit to a mask or to a lead in tube to the mask to deliver respiratory gases to the user. The illustrated components of the respiratory interface are a seal module 101 and elbow 102. The seal module 101 and elbow 102 may have the features and operation of earlier described embodiments, except the differences described below.

Figures 38a and 38b are schematic views of magnet parts of the seal module 101 and elbow 102 in incorrect and correct orientations relative to one another respectively. When the seal module 101 and the elbow 102 are correctly oriented (Figure 38b), the outlet aperture 111 of the elbow and the inlet aperture 110 of the seal module are aligned. As shown in Figure 38b this involves alignment of the apertures' 110, 111 central axis. Figure 38a shows the misalignment of the apertures' 110, 111 central axis when the seal module and the elbow are incorrectly oriented.

The magnetic coupling system 450 comprises two annular magnets 451, 453 provided in the seal module 101 with a space between them. One magnet is a coupling magnet 453 and one magnet is a repulsing magnet 451. Both magnets 451, 453 surround and are spaced from the inlet aperture 110 of the seal module 101. The magnetic coupling system 450 also comprises an annular magnet 455 in the elbow 102 in a location such that it will align with the coupling magnet 453 on the seal module 101 when the elbow is correctly orientated with respect to the seal module. In the embodiment shown, the elbow 102 has a flexible flange 457 and the annular magnet 455 is provided at or near a periphery of the flexible flange 457. The flange 457 extends outwardly from the elbow 102 and extends around the perimeter of the elbow. Although it is to be appreciated that in other embodiments the flange only extends around a portion of the elbow or comprises a plurality of flange segments that are distributed about the perimeter of the elbow. The flange 457 is located close to the outlet aperture 111 of the elbow. The flange 457 is configured to be positioned over a portion of the seal module's external surface surrounding the inlet aperture 110. The flexibility of the flange enables the flange to conform to the shape of the seal module's external surface and form a seal.

Although in the illustrated embodiments of Figures 36 - 38 the magnets 451, 453, 455 are shown as unitary annular magnets, in other embodiments one or more of the magnets comprises two or more magnets in an annular arrangement. Any or all of the annular magnets 451, 453, 455 can be made from a soft magnetic material, such that it is conformable to the geometry of the seal module 101. A soft magnet may provide an airtight connection between the seal module 101 and the elbow 102.

The coupling magnet 453 in the seal module 101 has an opposite pole (for example north) to the magnet 455 of the elbow 102 (for example south). The repulsing magnet 451 will have the same pole as the magnet 455 of the elbow 102 (for example south). Thus, the seal module 101 and elbow 102 will attract only in one (correct) orientation and/or position of the seal module 101 relative to the elbow, as illustrated by Figures 38a - incorrect orientation, and 38b - correct orientation. Further, when in the incorrect orientation, the magnets 451, 453, 455 will encourage the elbow 102 to move towards the correct orientation and/or position. This will occur if the elbow 102 is correctly aligned and is then moved or knocked. That is, the elbow 102 is self-centring or self-locating. If a user places the elbow 102 near the seal module 101, the magnets 451, 453, 455 will encourage the elbow 102 towards the correct orientation.

As described above in relation to other embodiments, interfaces of the disclosure may rely solely on magnetic attraction of the seal module 101 to the elbow 102. In alternative embodiments a magnetic coupling system supplements, and is provided in addition to, other coupling systems as described above. In some embodiments, the interface relies predominantly on mechanical coupling to hold the seal module 101 relative to the elbow 102, and a magnetic coupling system as described is provided primarily to self-centre the seal module 101.

### Magnet modular headgear

Figure 15 schematically shows an embodiment of headgear for a respiratory interface, comprising headgear parts and a magnetic coupling system arranged to magnetically couple headgear parts to one another.

In the embodiment shown the headgear comprises a rear strap 500 and top or crown strap 501, which couple magnetically to the ends of left and right side straps or side arms 503 from mask 504, which extend in use across the left and right sides of the face of the wearer. In the embodiment shown landing parts 503a are provided at the ends of side straps or arms 503.The left and right ends of rear strap 500 comprise a magnet or magnets and the left and right ends of top strap 501 each comprise a magnet or magnets, as do the landing parts 503a of left and right side straps or arms 503, so that the ends of the rear strap 500 and top strap 501 can be coupled to the ends 503a of the side straps or arms 503, to assemble the headgear for use as shown.

In embodiments the first and second, and optionally third or third and fourth headgear parts, comprise at least one magnet at or towards one end thereof and at least one ferrous part at or towards another end thereof.

The magnets may be discrete solid magnets or comprise embedded particulate magnetic material.

Headgear in any form such as bifurcated or four point headgear having both top and bottom side straps to the interface on left and right sides, may be formed in whole or part as described so that for example headgear may have a top strap which is attached to the rest of the headgear magnetically and can be removed if desired by the user.

In embodiments at least two magnets associated with one of one of the first and second, and optionally third or third and fourth headgear parts and another with another of the first and second, and optionally third or third and fourth headgear parts, are provided to the headgear parts such that they attract when the headgear parts are coupled in a predetermined correct orientation and repel in any orientation other than said predetermined correct orientation.

### Headgear buckle (also headgear to mask buckle)

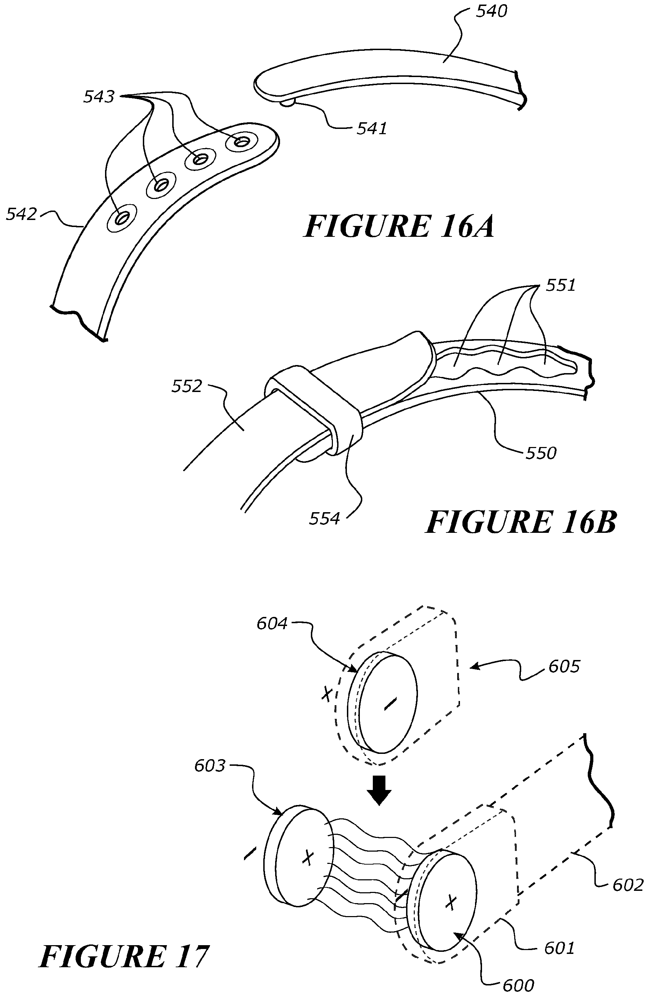

Figures 16A and 16B show embodiments of headgear for a respiratory interface comprising a magnetic coupling system to magnetically couple headgear straps or parts together.

In Figure 16A straps 540 and 542 adjustably connect together. Strap 540 carries protrusion 541 having a magnet or magnets associated therewith such as embedded in the strap at and within the protrusion during moulding of a plastic strap for example, or embedded in a separate plastic part in turn bonded to a strap of soft material strap such as Breath-O-Prene^{®} or similar material strap for example. Strap 542 carries a series of adjacent apertures 543 each having a small annular magnet embedded in the strap around the aperture. The magnet in protrusion 541 and associated with apertures 543 are oriented such that when the protrusion 541 is entered into one of the apertures 543 it will magnetically attract to and couple to the strap.

Figure 16B shows a similar embodiment in which protrusion 553 is provided on the underside of the end of strap 552, and recesses 551 which are optionally semi-connected to form a channel with corrugated sides as shown, are provided in the end of strap 550.Whereas in the embodiment of Figure 16A the apertures 543 may penetrate completely through the strap end, in the embodiment of Figure 16B the apertures 551 are blind and magnet or magnetic particulate material alternatively are embedded during plastic moulding of the strap for example in the base of the apertures 551.This embodiment also comprises a loop 554 through which the strap end 552 passes before connecting to strap 550.

Alternatively the magnetic protrusion 541 or 553 may be provided at or towards the end of a side strap which couples to a mask or forehead rest part of a mask, and apertures 543 or 551 provided on the left and right sides of the mask body or forehead rest, to couple the side straps to the mask body or forehead rest in the same way.

In embodiments the first headgear part comprises at least one magnet or at least one ferrous part associated with said protrusion and the second headgear part or mask comprises at least one magnet or at least one ferrous part associated with said one or more recesses. In embodiments the headgear comprises the first headgear part comprises at least one magnet associated with said protrusion and the second headgear part or mask comprises at least one magnet associated with said one or more recesses.

### Headgear connection broken by 3rd magnet

Figure 17 shows an embodiment of headgear for a respiratory interface comprising a magnetic coupling system, and a decoupling tool.

In the embodiment shown magnet 600 is provided at or towards the end 601 of strap 602.For example magnet 600 may be a solid or particulate material magnet embedded within a plastic moulded strap end 600 of strap 602 which may otherwise be of a softer strap material for example. Magnet 603 is similarly embedded in another strap end or headgear part, or a mask body including forehead rest, for example. Decoupling tool 605 is provided, comprising solid or particulate material magnet 604 embedded therein. When the decoupling tool 605 is inserted between the strap ends or the strap and mask part, to decouple same, specifically in the example shown between magnet 600 and magnet 603, in the correct orientation, doing so will tend to force the strap ends or the strap and mask part, specifically in the example shown the magnets 600 and 603, apart decoupling the same. For example the decoupling tool 604 may be slid along the inside face of strap 602 and strap end 601 towards magnet 600 and 603 in use.

Preferably the decoupling tool 605 comprises a visual indicator on an exterior thereof indicating a correct orientation for inserting the decoupling tool 605 between the straps and mask or two straps. For example the decoupling tool may have a colour or word or shape embedded in or marked on the surface of one or both sides, such as 'inside' and 'outside' or similar, or the decoupling tool may be formed in a shape which indicates to a user the correct orientation relative to the parts being decoupled in which the tool should be used. In a further embodiment the decoupling tool 605 may have a tapered/sharp leading edge in order to initially prise apart coupled magnets, with decoupling of magnets 600 and 603 further assisted by an embedded magnet 604 as the tool is manually pushed therebetween.

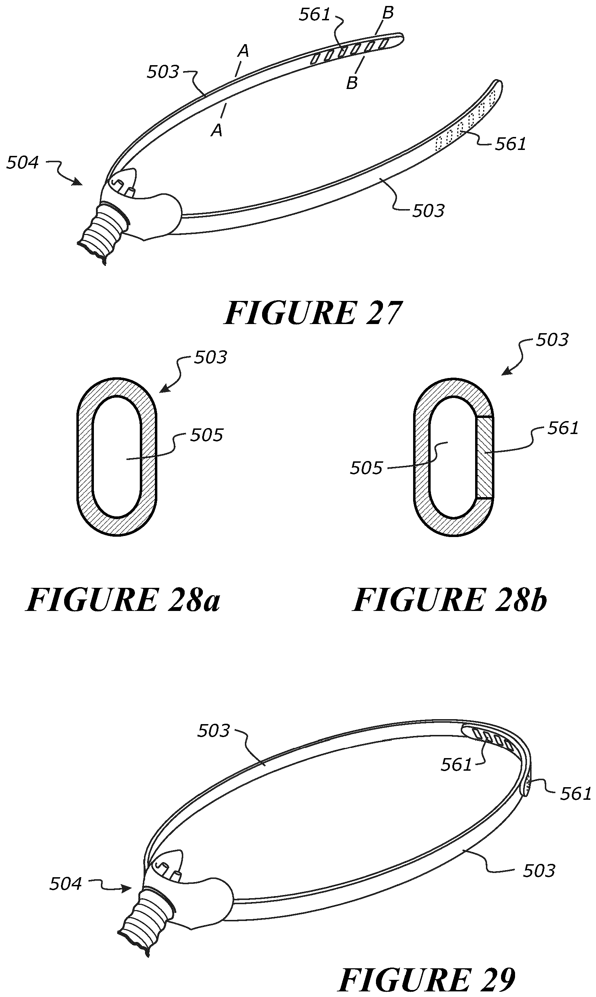

With reference to figures 27 to 29, another embodiment of headgear for a respiratory interface is shown. The interface may have the features and operation of earlier described embodiments, except the differences described below. The headgear comprises a first headgear part having an inflatable portion, a second headgear part, a magnetic coupling system arranged to magnetically hold the first and second headgear parts together. The first headgear part and second headgear part are left and right side arms 503 respectively. The arms 503 are in the form of straps. The first left and right side arms 503 overlap when engaged.

Figure 28a shows a cross-section of an arm 503 that is free of magnetic material. Figure 28b having a magnet 561. The left and right side arms 503 both comprise magnet free portions and portions with one or more magnets 561. As described in relation to other embodiments, each magnet 561 may be a solid or particulate material magnet embedded within a plastic moulded strap end of the strap which may otherwise be of a softer strap material for example.

The headgear has a decoupling mechanism that moves the arms and decouples the magnets. The decoupling mechanism comprises a pump associated with the inflatable portion that is arranged such that activation of the pump causes the inflatable portion to inflate, causing the first and second parts to move relative to each other. The pump is a squeeze pump that causes the inflatable portion to inflate by being squeezed by a user. The pump is preferably formed as part of the mask 504, but may be provided as part of the arms 503 or other part of the interface or headgear.

In the embodiment shown, the left and right side arms 503 both have an inflatable portion and activation of the pump causes the inflatable portion of both arms to inflate. The arms 503 move away from each other by one or both of the arms moving from a curved configuration (figure 29) to a relatively straight configuration (figure 27). In the embodiment shown, a substantial portion or the entire length of each or both of the arms 503 is inflatable. As shown in Figures 28a and 28b the inflatable portion of the arm(s) is provided by a hollow core 505 formed in the arms.

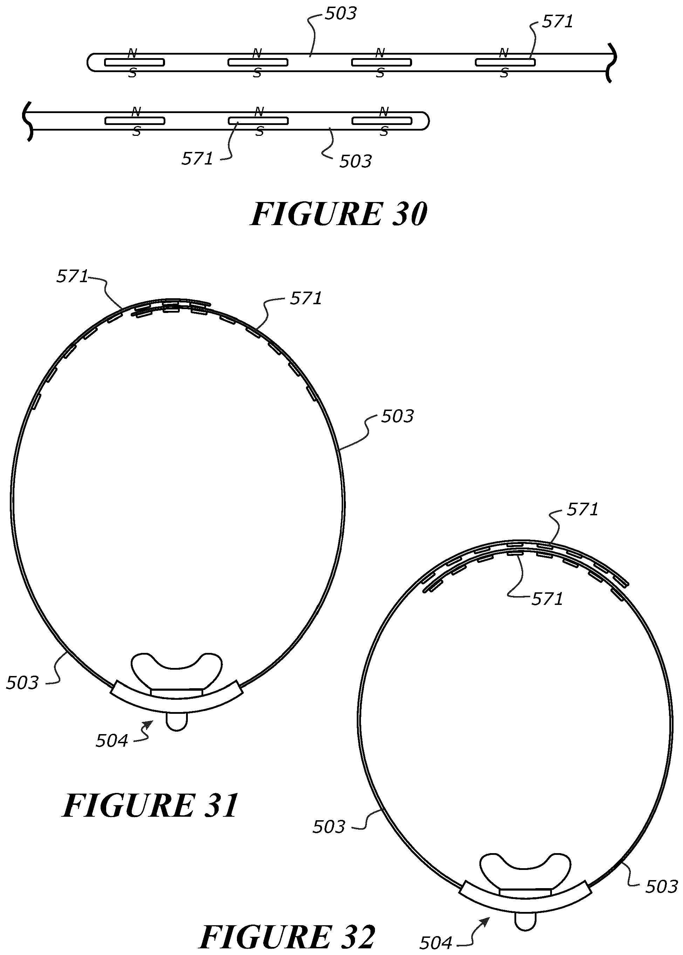

With reference to figures 30 to 32, another embodiment of headgear for a respiratory interface is shown. The interface may have the features and operation of earlier described embodiments, except the differences described below. In Figure 31 and 32, the arms 503 adjustably connect together. Both straps have magnets 571 associated therewith such as embedded in the arms 503 during moulding of a plastic strap for example, or embedded in a separate plastic part in turn bonded to a strap of soft material strap such as Breath-O-Prene^{®} or similar material strap for example. The magnets 571 in each arm 503 are oriented such that when the straps overlap, they will magnetically attract to each other. In alternative embodiments, one of the arms 503 has magnets and the other strap has at least one ferrous portion. Figure 31 is a schematic view of the headgear of figure 30 in a relatively large sized configuration, and figure 32 is a schematic view of the headgear of figure 30 in a relatively small sized, in-use configuration.

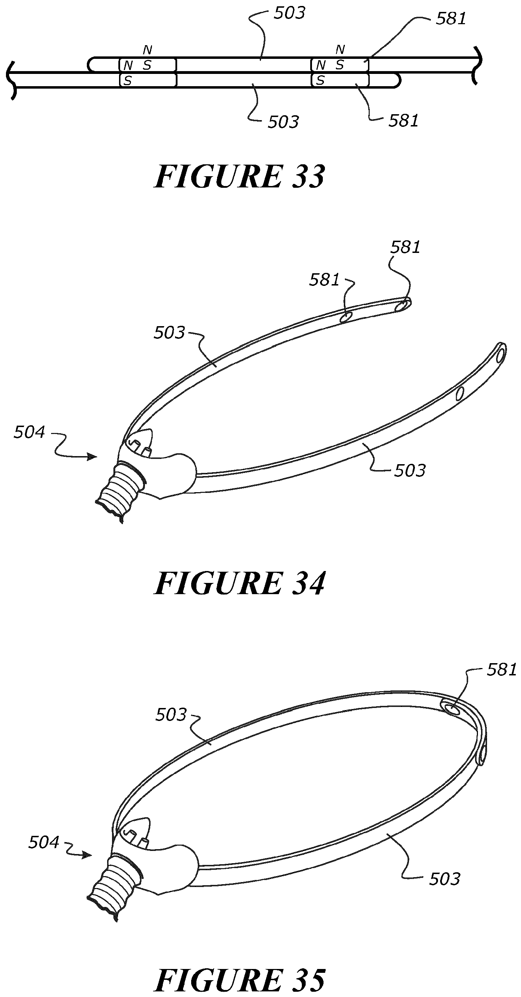

With reference to figures 33 to 35, another embodiment of headgear for a respiratory interface is shown. This embodiment is similar to the embodiment shown and described in relation to figures 30 to 32, except that the size of the strap is personalised to the user. In particular, the user's head will be measured and the straps will be manufactured to have the magnets 581 positioned in the correct location to fit the user.

### Further disclosure regarding headgear

In some configurations, portions of the headgear assembly can be substantially non-stretchable. For example, the back strap, top strap and upright straps can be non-stretchable such that the portion that encircles the occipital region of the users head is made from non-stretch material. In some configurations, non-stretchable portions can comprise of a substantially non-stretch insert. The non-stretch insert can be attached to the headgear assembly, for example, by over-lock stitching, by ultrasonic welding, by use of glue or other adhesives, or by any other method. In some configurations, one or more of the side straps can also be formed of a substantially inelastic or non-stretchable material. The side straps can be formed of a semi-rigid, self-supporting material such that the semi-rigid headgear assembly can assume a substantially three-dimensional shape and generally does not tangle. The non-stretchable characteristic can be achieved by embedding at least one relatively inelastic panel in the portion of the headgear assembly that is desirably non-stretchable. The panel can be formed of a relatively low-stretch material, such as a polyester Breath-O-Prene^{®} material, for example but without limitation. In some configurations, the headgear can be semi-rigid to secure the mask assembly to the user's head. The semi-rigid headgear can be formed as a composite structure comprising a semi-rigid strap that is joined to a soft edging. For example, the soft edging can be bonded to the semi-rigid strap by plastic overmolding or by use of an adhesive. The soft edging can be butt-joined to the semi-rigid strap, without the soft edging overlapping the semi-rigid strap, to maintain the continuous profile of the semi-rigid headgear. The semi-rigid strap can define and maintain the semi-rigid headgear shape as tension is applied from the straps to pull the mask assembly towards the user's head. In other words, the semi-rigid strap can be sufficiently rigid along its planar axis to prevent its upper and lower side straps from overly deforming under tension. The semi-rigid strap can be made from a variety of rigid or semi-rigid materials, including plastic or metal. In some configurations, the semi-rigid strap is made from PVC. Especially in connection with a semi-rigid headgear assembly, it has been found that the shape holding, or self-supporting nature, can result in an overall assembly that is intuitive to fit. In particular, where the connection and/or headgear members are self-supporting such that they maintain a three-dimensional form as discussed earlier, the headgear can be fitted in the correct orientation with very little if any instruction. In a self-supporting arrangement, the tendency of the straps to not tangle also reduces the time taken to fit the overall assembly. As used herein, the term "semi-rigid" is used to denote that the headgear assembly is sufficiently stiff such that the headgear assembly can assume a three-dimensional shape with dimensions approximating the head of the user for which the headgear is designed to fit while also being sufficiently flexible to generally conform to the anatomy of the user. For example, some of the other components (e.g., side straps) of the headgear assembly may also be partially or wholly "semi-rigid" such that the components are capable of holding a three-dimensional form that is substantially self-supporting. A "semi-rigid" headgear assembly is not intended to mean that each and every component of the headgear assembly is necessarily semi-rigid. For example, the substantially three-dimensional form that the self-supporting headgear assembly may assume may relate primarily to the rear and top portions of the headgear assembly. In addition, the semi-rigid headgear assembly may include semi-rigid regions that extend forward of the ears and above the ears when placed on the head of the patient.

The upper and lower side straps can be formed of a semi-rigid material, as well. Where used herein, the semi-rigid materials can include molded plastic or sheet materials that include, but are not limited to, homogeneous plastic materials and bonded non-woven fiber materials. In some configurations, the semi-rigid properties of the materials can be achieved with high-density foam material. The dense foam material can provide some structural rigidity to the upper and lower side straps, or other portions of the headgear assembly. In some configurations, the semi-rigid material can include textiles that are semi-rigid, such as denim or hemp. In some configurations, the material can comprise a laminate structure of both conformable and semi-rigid portions, for example but without limitation. The semi-rigid straps may be of a self-supporting, resilient, substantially inelastic material, such as Santoprene, polyolefin, polypropylene, polyethylene, foamed polyolefin, nylon or non-woven polymer material for example but without limitation. In some configurations, the semi-rigid strap is formed from the polyethylene or polypropylene families. The semi-rigid strap can be formed of a material such that the semi-rigid headgear is substantially shape-sustaining under its own weight regardless of its orientation. A soft edging can cover or attach to at least a portion of the periphery of the semi-rigid strap. In some configurations, the soft edging does not cover the front or rear faces of the semi-rigid strap and is instead attached adjacent to the edge of the semi-rigid strap. For example, the thicknesses of the soft edging and semi-rigid strap can be the same at the location where they are joined together. As used herein with respect to headgear and straps, "soft" is used to describe a hand of the material, which means the quality of the material assessed by the reaction obtained from the sense touch. In addition, as used herein with respect to headgear and straps, "conformable" is used to describe the ability of the material to conform to the anatomical features of the patient (e.g., around a facial feature). In particular, a strap including at least an element of "soft" and/or "conformable" material also may be "semi-rigid" and/or axially "non-stretchable." The soft edging can have a uniform thickness, or in some configurations, an uneven thickness. For example, in some configurations the soft edging is the same thickness as the semi-rigid strap. In other configurations, the soft edging is thinner than the semi-rigid strap, forms a bulbous end to the semi-rigid strap, or is simply thicker than the semi-rigid strap. Any one particular soft edging thickness and shape can apply to a portion or the entire semi-rigid strap, or may be combined with any other particular covering thickness and shape. Many other thickness configurations may be provided, as well. In addition, material thickness may be symmetrically or asymmetrically applied to the semi-rigid strap. For example, in some configurations the thickness of either end the soft edging is symmetrically applied to the semi-rigid strap. In some configurations the semi-rigid strap is selectively thickened to provide extra rigidity and support. Finally, in some configurations, venting through-holes are provided throughout the semi-rigid headgear (such as on the semi-rigid strap or on soft edging) to provide ventilation and sweat management.

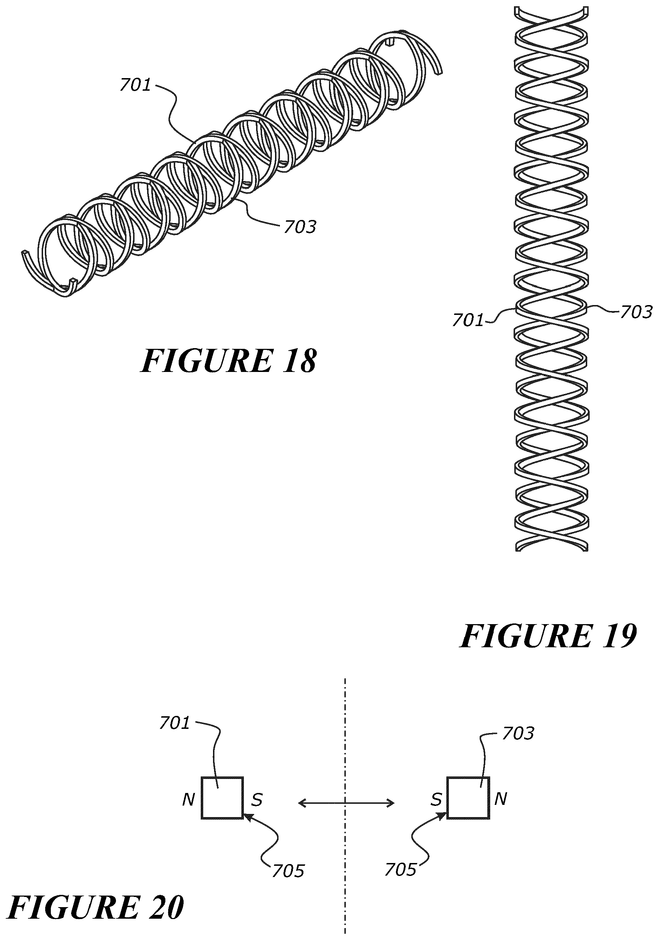

With reference to figures 18 to 22, a magnetic repulsing system is shown that is adapted to be incorporated into a conduit for delivering respiratory gases to a user. The conduit is connectable to a mask or to a lead in tube to the mask and comprises an inlet, an outlet, and an enclosing wall defining at least one gases passageway between said inlet and said outlet. The magnetic repulsing system is incorporated into the enclosing wall to magnetically repulse a portion of the wall from a diametrically opposite portion of the wall. In particular, the magnetic repulsing system comprises one or more magnets that are spiral or helically shaped magnets. The embodiment of figures 18 to 22 has two spiral or helically shaped magnets of which one spirals in a clockwise direction 701 and the other spirals in a counterclockwise direction 703. In an alternative embodiment, the magnetic repulsing system comprises a single spiral or helically shaped magnet. The inner surfaces 705 of the magnets that face each other are the same pole and repel each other. As a consequence, the repulsion prevents or at least substantially inhibits the conduit from being crushed or compressed.

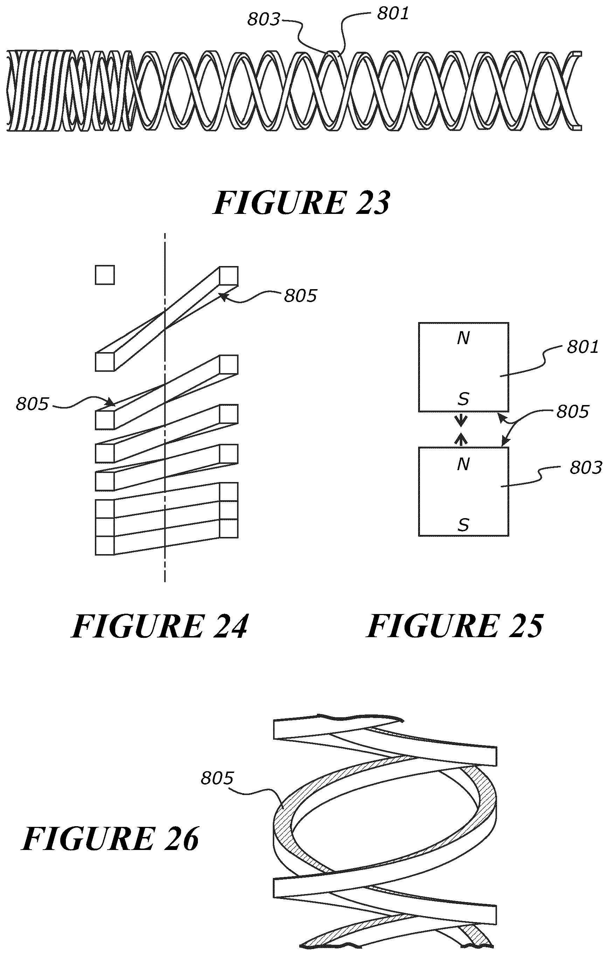

With reference to figures 23 to 26, a magnetic system according to another embodiment that is adapted to be incorporated into a conduit for delivering respiratory gases to a user, is shown. The magnetic system is arranged to magnetically collect, i.e. automatically retract and/or retain, a portion of a length of an enclosing wall of the conduit. In particular, the adjacent surfaces 805 of the magnets are the opposite poles and attract each other. As a consequence, the magnet collects a length of the conduit into a shorter length. The conduit may stretch, fold, or gather between the longer and shorter lengths. As the conduit, or a portion of the conduit, is stretched or pulled, the adjacent magnet portions will initially work against the pulling force and maintain the conduit in the shorter length. However, as the pulling force is increased, the magnetic force will be overcome and the conduit, or portion of the conduit, will have the longer length. Figure 24 shows a portion of conduit in which the lower portion is collected by the magnetic coupling system and the upper portion is configured such that the adjacent magnetic portions are spaced at a distance that is greater than their fields reach so the conduit is not collected.

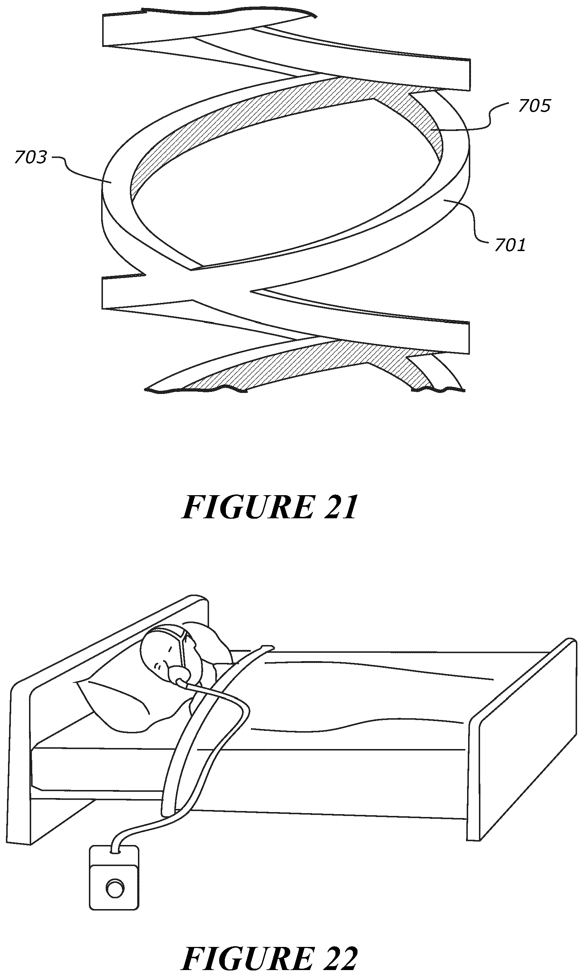

For the embodiments described above in relation to figures 18 to 26, the magnets may comprise a segment of solid (non-particulate) magnetic material or may comprise particulate magnetic material. The particulate magnetic is material embedded in a part of the conduit. For example, the particulate magnetic material may be integrally formed with the enclosed wall, such as during an extrusion process. The magnets may extend along part of, substantially most of, or the entire length of the conduit. In addition, the magnets may extend around only part of the circumference of the conduit. The enclosing wall is preferably a soft and flexible material. The soft and flexible material is comfortable against the wearer's face, such as a silicone material. Furthermore, the magnets are preferably formed from soft magnetic material and thus provide the conduit with a soft and flexible feel for the patient. The material may also provide a grip to anchor or secure the hose to adjacent objects, such as a bed cover (Figure 22).

The foregoing describes the invention including preferred forms thereof and alterations and modifications as will be obvious to one skilled in the art are intended to be incorporated in the scope hereof. The invention is defined by the following claims.

## Claims

1. A respiratory interface comprising:
a first seal part (300) configured to interface to a user's mouth to deliver respiratory gases to the user, and
a second seal part (302) configured to interface to the user's nose to also deliver respiratory gases to the user, and
a magnetic coupling system arranged to magnetically couple the first seal part (300) and the second seal part (302) to one another.

2. The respiratory interface of claim 1, wherein the one or both of the seal parts (300,302) are configured to magnetically couple to a common mask frame, and the one or other of the seal parts (300,302) may be detached to enable the respiratory interface to be used as an oral-only or nasal-only interface.

3. The respiratory interface of claim 1 or claim 2, wherein the magnetic coupling system comprises at least one magnet associated with one of the first seal part (300) and the second seal part (302) or common mask frame and at least one ferrous part associated with one of the first seal part (300) and the second seal part (302) or common mask frame.

4. The respiratory interface of claim 1 or claim 2, wherein the magnetic coupling system comprises magnets associated with each of the first seal part (300) and the second seal part (302) or one of the first seal part (300) and the second seal part (302) and the mask frame.

5. The respiratory interface of any one of claim 1 to 4, wherein the second seal part (302) is an indirect nasal seal.

6. The respiratory interface of any one of claim 1 to 4, wherein the second seal part (302) is a direct nasal seal.

7. The respiratory interface of any one of claims 1 to 6, wherein the first seal part (300) comprises magnets (303) around an upper peripheral part, and the second seal part (302) comprises magnets (304) around a lower peripheral part.

8. The respiratory interface of any one of claims 1 to 6, wherein the magnetic coupling system comprises a single strip magnet in each of the first and second seal parts (300,302).

9. The respiratory interface of any one of claims 1 to 6, wherein the magnetic coupling system comprises an embedded particulate magnetic material embedded in each of the first and second seal parts (300,302).

10. The respiratory interface of any one of claims 1 to 6, wherein the magnetic coupling system comprises an embedded particulate magnetic material embedded in a soft synthetic insert (350) in each of the first and second seal parts (300,302).

11. The respiratory interface of any one of claims 1 to 10, wherein the first seal part (300) and the second seal part (302) are configured to be attached or detached from one another, and the respiratory interface is configured to be used with both seal parts (300,302) together or with the first seal part (300) only.

12. The respiratory interface of any one of claims 1 to 10, wherein a conduit (301) is configured to be connected to the second seal part (302), and the respiratory interface is configured to be used with both seal parts (300,302) together or with the second seal part (302) alone.

13. The respiratory interface of any one of claims 1 to 12, wherein the magnetic coupling system comprises at least one magnet associated with one of first seal part (300), the second seal part (302), or the mask frame, and at least one magnet associated with another of the first seal part (300), the second seal part (302), or the mask frame, are provided to the seal parts (300,302) or mask frame such that they attract when the seal parts are coupled to each other or to the mask frame in a predetermined correct orientation and repel in any orientation other than said predetermined correct orientation.

14. The respiratory interface of any one of claims 1 to 13, wherein the respiratory interface further comprises a headgear (103) to hold the interface in position on a user's head when worn.

## Patentansprüche

1. Atemschnittstelle, umfassend:
ein erstes Dichtungsteil (300), das konfiguriert ist, um eine Schnittstelle zu einem Mund eines Benutzers zu bilden, um dem Benutzer Atemgase zuzuführen, und
ein zweites Dichtungsteil (302), das konfiguriert ist, um eine Schnittstelle zu einer Nase des Benutzers zu bilden, um dem Benutzer ebenfalls Atemgase zuzuführen, und
ein magnetisches Kopplungssystem, das angeordnet ist, um das erste Dichtungsteil (300) und das zweite Dichtungsteil (302) magnetisch miteinander zu koppeln.

2. Atemschnittstelle nach Anspruch 1, wobei das eine oder beide der Dichtungsteile (300,302) konfiguriert sind, um an einen gemeinsamen Maskenrahmen magnetisch gekoppelt zu werden, und das eine oder das andere der Dichtungsteile (300,302) gelöst werden kann, um zu ermöglichen, dass die Atemschnittstelle als reine Mund- oder reine Nasenschnittstelle verwendet wird.

3. Atemschnittstelle nach Anspruch 1 oder Anspruch 2, wobei das magnetische Kopplungssystem mindestens einen Magneten, der einem von dem ersten Dichtungsteil (300) und dem zweiten Dichtungsteil (302) oder dem gemeinsamen Maskenrahmen zugeordnet ist, und mindestens ein eisenhaltiges Teil, das einem von dem ersten Dichtungsteil (300) und dem zweiten Dichtungsteil (302) oder dem gemeinsamen Maskenrahmen zugeordnet ist, umfasst.

4. Atemschnittstelle nach Anspruch 1 oder 2, wobei das magnetische Kopplungssystem Magnete umfasst, die jeweils dem ersten Dichtungsteil (300) und dem zweiten Dichtungsteil (302) oder einem von dem ersten Dichtungsteil (300) und dem zweiten Dichtungsteil (302) und dem Maskenrahmen zugeordnet sind.

5. Atemschnittstelle nach einem der Ansprüche 1 bis 4, wobei das zweite Dichtungsteil (302) eine indirekte Nasendichtung ist.

6. Atemschnittstelle nach einem der Ansprüche 1 bis 4, wobei das zweite Dichtungsteil (302) eine direkte Nasendichtung ist.

7. Atemschnittstelle nach einem der Ansprüche 1 bis 6, wobei das erste Dichtungsteil (300) Magnete (303) um einen oberen Umfangsteil umfasst und das zweite Dichtungsteil (302) Magnete (304) um einen unteren Umfangsteil umfasst.

8. Atemschnittstelle nach einem der Ansprüche 1 bis 6, wobei das magnetische Kopplungssystem einen einzigen Streifenmagneten in jedem von dem ersten und dem zweiten Dichtungsteil (300, 302) umfasst.

9. Atemschnittstelle nach einem der Ansprüche 1 bis 6, wobei das magnetische Kopplungssystem ein eingebettetes partikelförmiges magnetisches Material umfasst, das in jedes von dem ersten und dem zweiten Dichtungsteil (300, 302) eingebettet ist.

10. Atemschnittstelle nach einem der Ansprüche 1 bis 6, wobei das magnetische Kopplungssystem ein eingebettetes partikelförmiges magnetisches Material umfasst, das in einen weichen synthetischen Einsatz (350) in jedem von dem ersten und dem zweiten Dichtungsteil (300,302) eingebettet ist.

11. Atemschnittstelle nach einem der Ansprüche 1 bis 10, wobei das erste Dichtungsteil (300) und das zweite Dichtungsteil (302) konfiguriert sind, um aneinander befestigt oder voneinander gelöst zu werden, und die Atemschnittstelle konfiguriert ist, um mit beiden Dichtungsteilen (300,302) zusammen oder nur mit dem ersten Dichtungsteil (300) verwendet zu werden.

12. Atemschnittstelle nach einem der Ansprüche 1 bis 10, wobei eine Leitung (301) konfiguriert ist, um mit dem zweiten Dichtungsteil (302) verbunden zu werden, und die Atemschnittstelle konfiguriert ist, um mit beiden Dichtungsteilen (300,302) zusammen oder mit dem zweiten Dichtungsteil (302) allein verwendet zu werden.

13. Atemschnittstelle nach einem der Ansprüche 1 bis 12, wobei das magnetische Kopplungssystem mindestens einen Magneten umfasst, der einem von dem ersten Dichtungsteil (300), dem zweiten Dichtungsteil (302) oder dem Maskenrahmen zugeordnet ist, und mindestens ein Magnet, der einem anderen von dem ersten Dichtungsteil (300), dem zweiten Dichtungsteil (302) oder dem Maskenrahmen zugeordnet ist, den Dichtungsteilen (300,302) oder dem Maskenrahmen derart bereitgestellt wird, dass sie sich anziehen, wenn die Dichtungsteile in einer vorbestimmten korrekten Ausrichtung aneinander oder an den Maskenrahmen gekoppelt sind, und sich in einer beliebigen anderen Ausrichtung als der vorbestimmten korrekten Ausrichtung abstoßen.

14. Atemschnittstelle nach einem der Ansprüche 1 bis 13, wobei die Atemschnittstelle ferner eine Kopfbedeckung (103) umfasst, um die Schnittstelle auf dem Kopf eines Benutzers in Position zu halten, wenn sie getragen wird.

## Revendications

1. Interface respiratoire comprenant :
une première partie d'étanchéité (300) conçue pour assurer l'interface avec la bouche d'un utilisateur pour délivrer des gaz respiratoires à l'utilisateur, et
une seconde partie d'étanchéité (302) conçue pour assurer l'interface avec le nez de l'utilisateur pour délivrer également des gaz respiratoires à l'utilisateur, et
un système d'accouplement magnétique agencé pour accoupler magnétiquement la première partie d'étanchéité (300) et la seconde partie d'étanchéité (302) l'une à l'autre.

2. Interface respiratoire selon la revendication 1, dans laquelle l'une et/ou l'autre des parties d'étanchéité (300, 302) sont conçues pour s'accoupler magnétiquement à un cadre de masque commun, et l'une ou l'autre des parties d'étanchéité (300, 302) peut être détachée pour permettre à l'interface respiratoire d'être utilisée en tant qu'interface buccale uniquement ou nasale uniquement.

3. Interface respiratoire selon la revendication 1 ou la revendication 2, dans laquelle le système d'accouplement magnétique comprend au moins un aimant associé à l'une de la première partie d'étanchéité (300) et de la seconde partie d'étanchéité (302) ou à un cadre de masque commun et au moins une partie ferreuse associée à l'une de la première partie d'étanchéité (300) et de la seconde partie d'étanchéité (302) ou au cadre de masque commun.

4. Interface respiratoire selon la revendication 1 ou la revendication 2, dans laquelle le système d'accouplement magnétique comprend des aimants associés à chacune de la première partie d'étanchéité (300) et de la seconde partie d'étanchéité (302) ou à l'une de la première partie d'étanchéité (300) et de la seconde partie d'étanchéité (302) et au cadre de masque.

5. Interface respiratoire selon l'une quelconque parmi la revendication 1 à 4, dans laquelle la seconde partie d'étanchéité (302) est un joint d'étanchéité nasal indirect.

6. Interface respiratoire selon l'une quelconque parmi la revendication 1 à 4, dans laquelle la seconde partie d'étanchéité (302) est un joint d'étanchéité nasal direct.

7. Interface respiratoire selon l'une quelconque des revendications 1 à 6, dans laquelle la première partie d'étanchéité (300) comprend des aimants (303) autour d'une partie périphérique supérieure, et la seconde partie d'étanchéité (302) comprend des aimants (304) autour d'une partie périphérique inférieure.

8. Interface respiratoire selon l'une quelconque des revendications 1 à 6, dans laquelle le système d'accouplement magnétique comprend un unique aimant en bande dans chacune des première et seconde parties d'étanchéité (300, 302).

9. Interface respiratoire selon l'une quelconque des revendications 1 à 6, dans laquelle le système d'accouplement magnétique comprend un matériau magnétique particulaire intégré dans chacune des première et seconde parties d'étanchéité (300, 302).

10. Interface respiratoire selon l'une quelconque des revendications 1 à 6, dans laquelle le système d'accouplement magnétique comprend un matériau magnétique particulaire intégré qui est intégré dans un insert synthétique mou (350) dans chacune des première et seconde parties d'étanchéité (300, 302).

11. Interface respiratoire selon l'une quelconque des revendications 1 à 10, dans laquelle la première partie d'étanchéité (300) et la seconde partie d'étanchéité (302) sont conçues pour être attachées ou détachées l'une de l'autre, et l'interface respiratoire est conçue pour être utilisée avec l'une et l'autre des parties d'étanchéité (300, 302) ensemble ou avec la première partie d'étanchéité (300) uniquement.

12. Interface respiratoire selon l'une quelconque des revendications 1 à 10, dans laquelle un conduit (301) est conçu pour être raccordé à la seconde partie d'étanchéité (302), et l'interface respiratoire est conçue pour être utilisée avec l'une et l'autre des parties d'étanchéité (300, 302) ensemble ou avec la seconde partie d'étanchéité (302) seule.

13. Interface respiratoire selon l'une quelconque des revendications 1 à 12, dans laquelle le système d'accouplement magnétique comprend au moins un aimant associé à l'un de la première partie d'étanchéité (300), de la seconde partie d'étanchéité (302) ou du cadre de masque, et au moins un aimant associé à un autre de la première partie d'étanchéité (300), de la seconde partie d'étanchéité (302) ou du cadre de masque, qui sont fournis aux parties d'étanchéité (300, 302) ou au cadre de masque de telle sorte qu'ils s'attirent lorsque les parties d'étanchéité sont accouplées l'une à l'autre ou au cadre de masque dans une orientation correcte prédéterminée et se repoussent dans l'une quelconque orientation autre que ladite orientation correcte prédéterminée.

14. Interface respiratoire selon l'une quelconque des revendications 1 à 13, dans laquelle l'interface respiratoire comprend en outre un équipement de tête (103) pour maintenir l'interface en position sur la tête d'un utilisateur lorsqu'elle est portée.
